(19) Europäisches Patentamt
European Patent Office
Office européen des brevets

(11) **EP 3 402 409 B1**

(12) **EUROPEAN PATENT SPECIFICATION**

(45) Date of publication and mention
of the grant of the patent:
**28.02.2024 Bulletin 2024/09**

(21) Application number: **17739110.9**

(22) Date of filing: **13.01.2017**

(51) International Patent Classification (IPC):
*A61B 6/00* *(2024.01)*          *G06V 10/24* *(2022.01)*
*G16H 20/40* *(2018.01)*          *G16H 30/40* *(2018.01)*
*G16H 40/63* *(2018.01)*          *A61B 6/12* *(2006.01)*
*G06F 18/22* *(2023.01)*

(52) Cooperative Patent Classification (CPC):
**A61B 6/505; G06V 10/24; G16H 20/40;
G16H 30/40; G16H 40/63;** A61B 6/12; G06F 18/22;
G06V 2201/033

(86) International application number:
**PCT/US2017/013572**

(87) International publication number:
**WO 2017/124043 (20.07.2017 Gazette 2017/29)**

(54) **SYSTEMS AND METHODS FOR INTRA-OPERATIVE IMAGE ANALYSIS**

SYSTEME UND VERFAHREN ZUR INTRAOPERATIVEN BILDANALYSE

SYSTÈMES ET PROCÉDÉS POUR ANALYSE D'IMAGE PEROPÉRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2016 US 201614995057
23.09.2016 US 201662399122 P**

(43) Date of publication of application:
**21.11.2018 Bulletin 2018/47**

(73) Proprietor: **DePuy Synthes Products, Inc.
Raynham, MA 02767 (US)**

(72) Inventors:
• **WOLLOWICK, Noah D.
Westport, CT 06880 (US)**
• **COOPER, Andrew, J.
Largo, FL 33770 (US)**
• **ALBERT, Cameron
Henrico, VA 23238 (US)**

(74) Representative: **Carpmaels & Ransford LLP
One Southampton Row
London WC1B 5HA (GB)**

(56) References cited:
WO-A1-2014/127354          US-A1- 2006 293 614
US-A1- 2013 197 687        US-A1- 2015 238 271
US-B1- 6 205 411           US-B2- 8 917 290

**Description**

**FIELD OF THE INVENTION**

The invention relates to systems and methods for intraoperative image analysis.

**BACKGROUND OF THE INVENTION**

Orthopaedic surgeons often analyse leg length discrepancies before hip replacement surgery by analysing radiographic images of an AP pelvis using a preoperative planning software system such as Medstrat™, Traumacad™, or Orthoview™. Image-based analysis systems also exist to analyse radiographic images intraoperatively to determine changes in leg length and offset. Existing preoperative and intraoperative analysis techniques for hip arthroplasty that use radiographic imaging for analysis do not automatically account and correct for femoral abduction when performing analysis.

The standard technique included in software systems for preoperative analysis of leg length discrepancy has been to use a leg length analysis tool that can be positioned so that a digital reference line transects similar anatomic points on both sides of the pelvis. Two vertical lines arise from the reference line and are positioned so that they are connected to similar anatomic points on the respective femurs. These femoral points are usually located on the lesser or greater trochanters.

There are a wide range of problems with this technique that make this analysis potentially unreliable. One major problem is that the technique doesn't account for femoral rotation and abduction differences between the operative and non-operative sides of the hip. These differences are often exacerbated by degenerative changes to the pathologic hip. This can lead to suboptimal outcomes during hip reconstruction because the surgeon may be trying to reconstruct leg length that was not properly quantified preoperatively.

Similarly, intraoperative analysis techniques that rely on radiographic imaging to overlay and compare preoperative and intraoperative images require either consistent positioning of the femur relative to the pelvis, or alternatively require the user to manually manipulate the images to account for differences. Either of these alternatives are timeconsuming and challenging processes with potential for suboptimal surgical outcomes due to incorrect analysis of leg length and/or offset in cases where femoral abduction has changed.

The challenge of accounting for differences in femoral positioning, everpresent in existing imaging-based analysis techniques for hip arthroplasty, could be solved by developing a system and method that automatically accounts and corrects for femoral abduction when performing either preoperative or intraoperative analysis.

It is therefore desirable to have a non-invasive system and method that provides image-based guidance and data while automatically correcting for any differences in femoral positioning.

WO 2014/127354 A1 discloses a system for positioning and adjusting the placement of an implant or instruments or bone fragment or reconstruction of anatomical bone architecture in a patient.

**SUMMARY OF THE INVENTION**

An object of the present invention is quantify features within images such as the positioning of bones imaged within a human, a mammal or other animal.

Another object of the present invention is to quantify restoration of orthopaedic functionality at a surgical site within a patient, even during a surgical procedure.

A further object of the present invention is to provide image analysis and feedback information suitable to enable better fracture reduction and/or optimal implant selection during the surgery.

Yet another object of the present invention is to capture and preserve a digital record of patient results for data collection and quality improvements in surgical procedures.

A still further object of the present invention is to improve the outcome of bone repositioning, fracture repair, and/or fixation within a patient.

The present invention provides a method according to claim 1 and a system according to claim 2. Embodiments are defined in the dependent claims.

**BRIEF DESCRIPTION OF THE DRAWINGS**

In what follows, preferred embodiments of the invention are explained in more detail with reference to the drawings, in which:

FIG. 1 is a schematic diagram of an Image Analysis System;

FIG. 2 is a Flowchart showing technique flow for preoperative leg length analysis comparing an operative side of a hip with the other, contralateral side of the hip;

FIG. 3 is a schematic screen view of a preoperative radiographic image of a bilateral AP pelvis with illustrated digital annotations used for scaling;

FIG. 4 is a schematic screen view of a digital point annotation placed on the greater trochanter on the ipsilateral hip in a preoperative image;

FIG. 5 is a schematic screen view with a digital circle annotation placed around the femoral head of the ipsilateral hip in a preoperative hip image;

FIG. 6 is a schematic screen view of a digital annotation placed along the longitudinal axis of the ipsilateral femur in the preoperative hip image;

FIG. 7 is a schematic screen view of a digital line annotation drawn between two identifiable points on the ipsilateral pelvic anatomy of the preoperative hip image;

FIG. 8 is a schematic screen view of a digital line annotation drawn between two identifiable points on the contralateral pelvic anatomy of the preoperative hip image;

FIG. 9 is a schematic screen view of a digital line annotation drawn between two identifiable points on the contralateral pelvic anatomy of the preoperative hip image;

FIG. 10 is a schematic screen view showing the operative hip from the original bilateral AP pelvis image overlaid with an inverted image of the contralateral hip, with the two images aligned and rotated according to drawn digital line annotations, with a femoral correction module, and with a display of the calculated discrepancy in preoperative leg length;

FIG. 11 is a flowchart showing the use of an 'Image Overlay' technique to calculate an intraoperative change in offset and leg length according to an aspect of the present invention;

FIG. 12 is an image of the right side of a patient's hip prior to an operation and showing a marker placed on the greater trochanter as a landmark or reference point;

FIG. 13 is an image showing a digital tool drawn in the femoral canal of (i) the pre-operative, ipsilateral femur or (ii) the contra-lateral femur, to identify the longitudinal axis of the femur;

FIG. 14 is a schematic screen view with a reference line drawn between two identifiable points on (i) the pre-operative, ipsilateral pelvic anatomy or (ii) the contra-lateral pelvic anatomy;

FIG. 15 is a schematic screen view indicating marking of the greater trochanter of the right-hand, intra-operative image as a femoral landmark;

FIG. 16 is a schematic screen view of two images, the left-hand image representing a pre-operative view and the right-hand image representing an intra-operative view with a circle placed around the acetabular component of an implant to enable scaling of that image;

FIG. 17 is an image showing a digital tool drawn in the femoral canal of the intraoperative femur to identify the longitudinal axis of the femur;

FIG. 18 is a schematic screen view with a reference line drawn on the intraoperative femur in the right-hand view;

FIG. 19 is an image with points marking the lowest point on the ischial tuberosity and points marking the obturator foramen and top of the pubic symphysis in both pre- and intra-operative views;

FIG. 20 is a schematic screen view of a section of the operative hip from the intraoperative image overlaid on the preoperative hip image according to the present invention;

FIG. 21 is an overlay image showing the intra-operative image superimposed and aligned with the pre-operative image according to the present invention;

FIG. 22 is a schematic diagram showing generation of a corrected landmark point and analysis of offset and length differential according to the present invention; and

FIG. 23 is a schematic screen view of a preoperative image and an intraoperative image positioned side by side with a grid and digital annotations to mark anatomic landmarks and other features on the images.

## DETAILED DESCRIPTION

Broadly, some techniques according to the present invention, referred to by the present inventors as "Image Overlay", place one image over another image during analysis to generate a combined overlapped image. Previous approaches for the "Image Overlay' technique made use of a pelvic reference line having two or more points to scale and align a preoperative image and an intraoperative image. The pelvic reference line having two or more points is also referred to as a "stationary base" as defined in U.S. Patent Application No. 14/630,300 filed 24 February 2015, sometimes referred to herein as "parent application", now US Publication No. 2015/0238271.

Alternative approaches for 'Image Overlay' technique according to the present invention obviate the need for the pelvic reference line or other stationary base. In some constructions, these alternatives instead rely upon certain image acquisition techniques, certain image manipulation techniques, certain known imaging information, and/or direct user manipulation to create consistent scale and alignment between (i) at least one of a first preoperative image and an inverted contralateral image and (ii) at least one of an intraoperative image and a second preoperative image.

Alternative constructions may alternatively apply absolute scaling to the preoperative and intraoperative images directly in each image, and without the need for reference lines to be drawn across stationary anatomy. For example, each image may be scaled by a ball marker or other scaling device, known magnification ratios of a radiographic device, or direct measurements of anatomical points (such as a direct measurement, via callipers, of the extracted femoral head, which can be used to scale the preoperative image).

Alternative constructions may also replace the 'stationary base' with various other techniques that could be used to scale and align the preoperative and intraoperative images relative to one another. One example of such a construction would involve overlaying two images and displaying them with some transparency so that they could both be viewed on top of one another. The user would then be prompted to rotate and change their sizing, so that the pelvic anatomy in the two images were overlaid as closely as possible.

Additionally, any change in positioning of the femur in the two images, relative to the pelvis, would adversely affect calculations in previous approaches of this technique. Maintaining femoral position while performing hip arthroplasty can pose a significant and often unrealistic challenge to a surgeon that is focused on performing a surgical procedure. Various approaches for the 'Image Overlay' technique according to the present invention can correct for deviations in femoral positioning between reference images (e.g. preoperative) and target images (e.g. intraoperative) by mathematically, and possibly visually (i.e. in a visually perceptible manner), correcting for any deviation in femoral position in at least one of the visual output and calculation output of offset and leg length.

Additionally, at least one identifiable anatomic "landmark", "stationary point" or "error point", or a set of landmarks stationary points or error points, may be selected and then designated as "digital annotations" on chosen images. The one or more landmarks, stationary points or error points are utilized in certain constructions to analyze the accuracy of the overlay process. This additional anatomic feature preferably is part of the stationary anatomy being anatomically compared. For example, the pelvic teardrop can be identified as an error point on the stationary anatomy. This anatomic feature can be used to depict any differences or errors in pelvic anatomy or the overlay which will enable the physician to validate, or to have more confidence in, the output of the present system. As generally utilized herein: (i) a "stationary point" refers to a point on a relatively stationary bone such as on the pelvis; (ii) a "landmark point" is located on an articulating bone such as a femur; (iii) an "error point" is preferably on pelvis and spaced from other points; and (iv) a "fixed point" is located on an implant, such as the shoulder of a femoral stem prosthesis.

The term "trial hip prosthetic" is utilized herein to designate an initial implant selected by a surgeon as a first medical device to insert at the surgical site, which is either the right side or the left side of a patient's hip in certain constructions. In some techniques, the trial prosthetic is selected based on initial digital templating similar to the procedure described the parent application.

The term "digital representation" or "digital annotation" as utilized herein includes a digital line having at least two points, e.g. a line representing a longitudinal axis or a diameter of an implant or a bone, or a digital circle or other geometric shape which can be aligned with an implant or a bone intraoperatively and then placed in a corresponding location in a preoperative image, or visa versa.

The term "vector" is utilised herein with the standard meaning of an Euclidean vector having an initial point or "origin" and a terminal point, representing magnitude and direction between the origin and the terminal point.

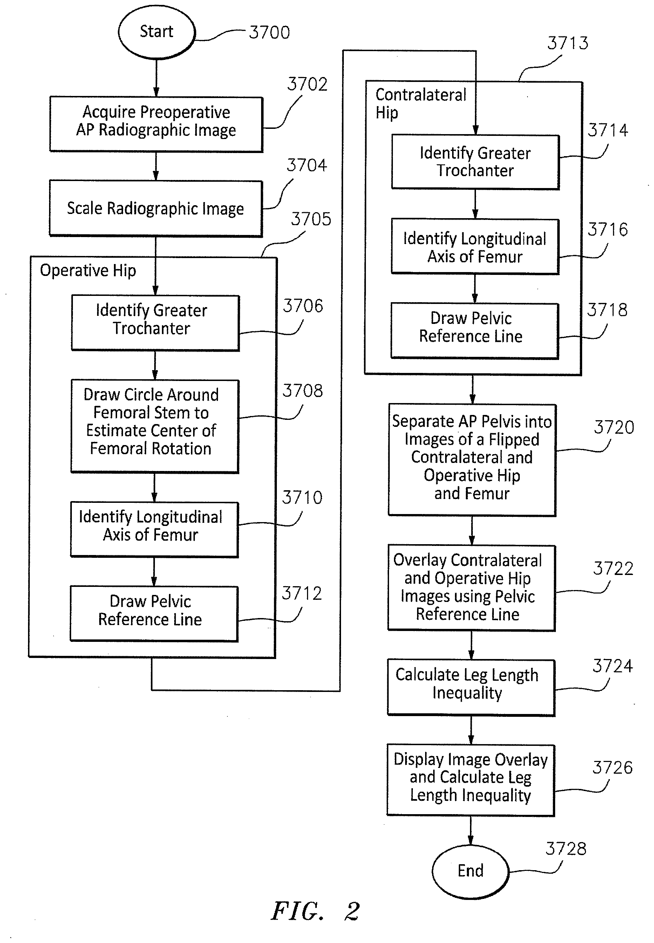

Software to accomplish the techniques described herein is located on a single computing device in some constructions and, in other constructions such as system 141, FIG. 2, is distributed among a server 155 and one or more user interface devices which are preferably portable or mobile. In some techniques a digitized X-ray image of the hip region of a patient along a frontal or anterior-to-posterior viewing angle is utilized for a screen view on a display and, in other techniques, a digital photograph "secondary" image of a "primary" X-ray image of the hip region of a patient along a frontal or anterior-to-posterior viewing angle is utilized for the screen view. In one construction, the screen view is shown on a computer monitor and, in another construction, is shown on the screen or viewing region of a tablet or other mobile computing device.

Moreover, if a computer interface tool, such as a stylus or light pen, is provided to the user in a sterile condition, than the user can remain within a sterile field of surgery while operating a computing device programmed according to the present invention.

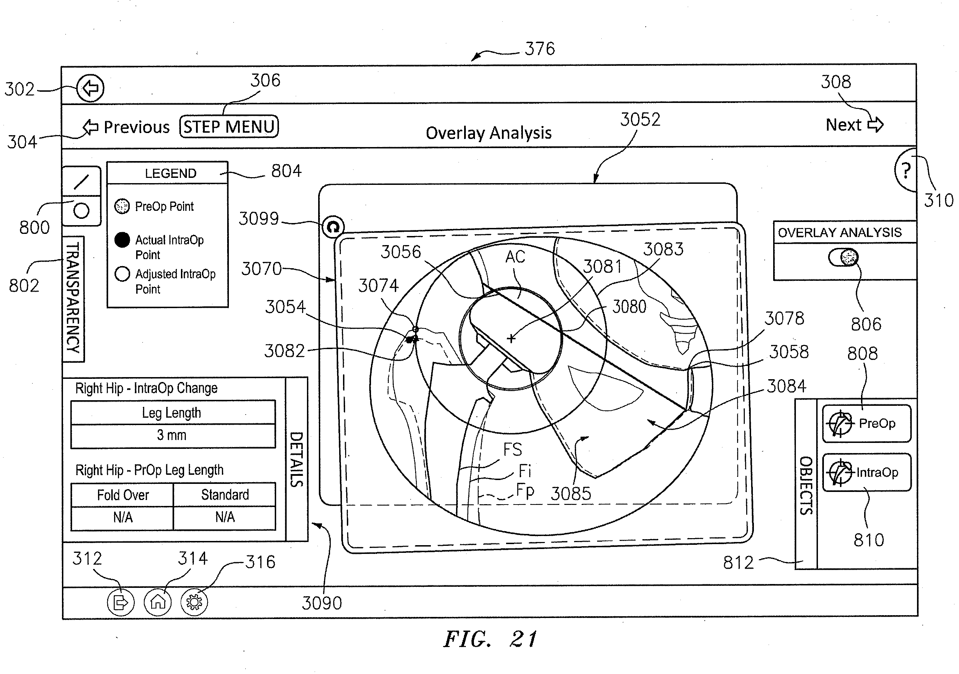

Hip- and femur-related constructions of the present system and method calculate intraoperative changes in offset and leg length using a reference image, also referred to as a "preop image", and an intraoperative image, also referred to as a "postop image" or an "intraop image". To accomplish this, one construction of the system requires two consistently scaled images that are overlaid and aligned according to the stationary anatomic region (such as the pelvis), the generation of at least one landmark point on the non-stationary, articulating anatomic region (such as the femur) in both images, a mechanism to identify the difference in femoral angle of the femur relative to the pelvis between the images, a mathematical correction module that adjusts for differences in the articulating femur in each image relative to the stationary pelvis and, finally, a calculation module that uses this input to calculate intraoperative changes in offset and leg length. As utilized herein, the term "femoral angle" refers to the orientation of the longitudinal axis of the femur relative to the pelvis; a "difference in femoral angle" is described in more detail below in relation to FIG. 21. The system may optionally include an error analysis module that identifies and analyses potential error in the system.

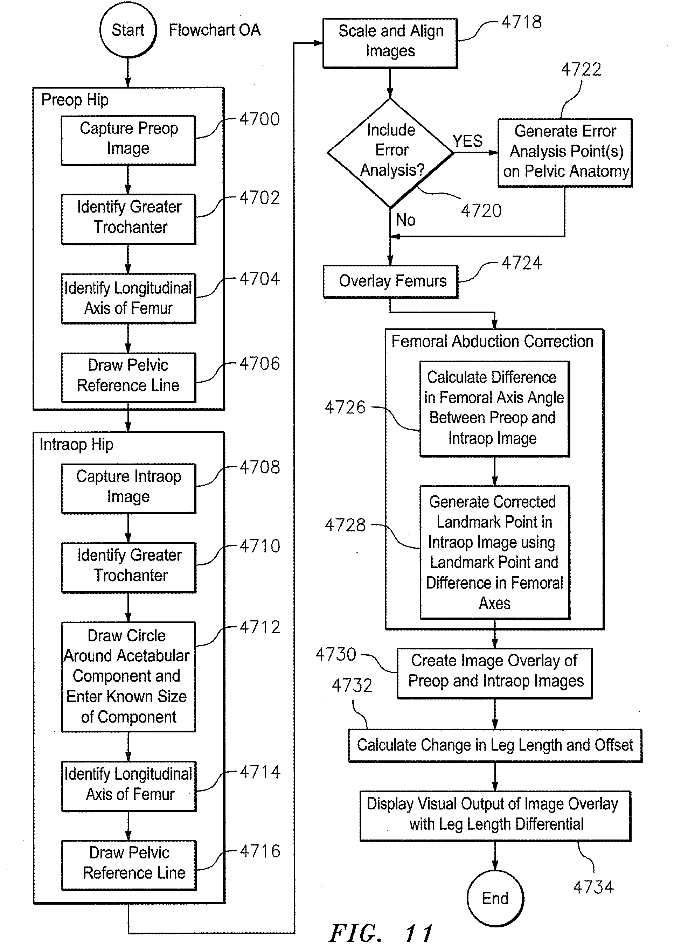

As described in more detail below in relation to FIGS. 11-21, an 'Image Overlay' process according to the present invention begins in some constructions by acquiring (i) at least one of a preoperative ipsilateral or an inverted contralateral image ("preop image" or "reference image"), and (ii) an intraoperative image ("intraop image"). The system generates at least one landmark point on the non-stationary femur in both images (such as identification of a consistent point on the greater trochanter in both images), generally performed with user guidance. Optionally, the system will generate at least one error point on the pelvis in both images to provide error analysis. If the images have not been previously scaled and aligned, the system will scale and align them using one of a plurality of techniques. One of the images is then overlaid according to the pelvic anatomy in both images.

In some constructions, the system identifies points that can be used to analyze possible error in the images relative to each other. The system additionally performs a series of steps to calculate any deviation in alignment of the non-stationary femur relative to the pelvic anatomy between the preop and intraop images. The system then creates an overlay of the preop and intraop image, taking into consideration and correcting for the effect of any difference in femoral angles between the two images as the system compares the relative position of the generated femoral landmark points. Finally, the system analyses the difference between the landmark points, including a correction for femoral alignment differences, and uses this data to calculate intraoperative change in offset and leg length.

Both preoperative and intraoperative analysis systems are disclosed. One example of this, wherein the example is not covered by the claimed invention, involves capture of a preoperative image of the bilateral AP pelvis to analyse at least one of leg length and offset of the hip to be operated on as compared to the contralateral hip prior to surgery, while also accounting for differences in femoral abduction between the legs relative to the pelvic anatomy. A preferred embodiment intraoperatively analyses at least one of leg length and offset of the operative hip compared to the preop hip (preop ipsilateral or contralateral), while also accounting for any movement corresponding to a change in femoral abduction. The preoperative analysis demonstrates the application of this system, while correcting for femoral abduction, while determining an intraoperative target for at least one of leg length and offset. Preoperative differences in femoral abduction can often be exacerbated by degenerative changes or pain of the deformed hip, making this femoral abduction correction critical to determining target measurements. Accounting for intraoperative change in femoral abduction is similarly critical when analysing actual surgical changes to measurements, because maintaining consistent position of the femur while the surgeon focuses on the mechanical aspects of the surgical operation is not a realistic goal. A mis-alignment of only several millimeters can have a noticeable impact on a patient.

The application of this system to preoperatively analyse leg length analysis techniques originates from the realization that present techniques are both inaccurate and inconsistent, and furthermore do not account for differences in femoral abduction. Traditional techniques generally involve drawing of a digital line that transects bilateral points on the pelvic anatomy. Then, two perpendicular lines, relative to this line, are drawn to similar points on the femoral anatomy, such as the greater trochanter or lesser trochanter. Using different femoral or pelvic points to perform this analysis, or varying amounts of femoral abduction when a radiographic image is taken, can often yield drastically different results.

Such uncertainty when using conventional techniques to preoperatively determine a leg length target is problematic for surgeons because they don't know the correct leg length target when performing hip arthroplasty. Surgeons often ignore preoperative leg length analysis for this reason and instead rely upon intraoperative radiographic images of the contralateral hip and the operative hip, with a trial prosthetic in place, to visually analyse patient leg length. This form of intraoperative analysis can be prone to visual analysis error and lead to suboptimal patient outcomes. This system and method that facilitates preoperative comparison using an overlay, as a replacement for conventional pre-operative techniques, can be used to provide a true leg length target for intraoperative decisionmaking.

A preferred example of this system which preoperatively analyzes a hip, wherein the example is not covered by the claimed invention, begins with capturing a bilateral image of an AP pelvis, which is then annotated with a series of digital annotations that enable the system to calculate preoperative difference in leg length between the ipsilateral and contralateral hip. Specifically, the system uses the digital annotations to separate the original image into a first image of the contralateral hip and femur, and a second image of the operative hip and femur. The system then flips one of these images so that the respective images are oriented in the same direction. Then, the system will make use of the digital annotations to render the images relative to one another and then calculate leg length inequality.

In a preferred implementation, the separated images will be rendered as an overlay, with the images placed on top of one another. This rendering will be preferably done with partially transparent images, so that each image can be viewed relative to one another in the overlay. The system generates the overlay of the two images by using a digital line annotation that connects similar points on the bony pelvic anatomy on both the contralateral and operative hips. The system will use these lines to perform some combination of operations that may include alignment, scaling, rotation and transformation. Conceptually, the system is using the digital line annotation drawn across pelvic anatomy to automatically align the images relative to one another.

This process of using a digital line annotation to automatically align the images will yield accurate results when there is strong pelvic symmetry and when the image is taken with the entire image of the pelvis in the AP plane. In reality, there are sometimes variations in pelvic symmetry between the contralateral and operative side, and it is often challenging to obtain an accurate preoperative AP image of the pelvis due to patient positioning, parallax, and other factors. Therefore, preferred constructions of the system may enable the user to manually adjust the overlay of the two images, so that the surgeon can modify the placement of the images in relation to one another, after the system auto-generates the overlay using the digital line annotations. Alternative constructions of this system may fully rely upon the user to overlay the pelvic anatomy of the two images instead of automatically orienting them according to a digital line annotation.

Note that alternative constructions will render the contralateral and ipsilateral images next to one another, instead of on an overlay, after transforming the images according to the pelvic overlay line annotation. While this alternative construction will work, it would limit a surgeon's ability to manually adjust the positioning of the images by eyeballing pelvic anatomy and potentially lead to increased error in the system analysis.

After the operative and contralateral images pelvis are overlaid by use of digital line annotations, the system makes use of digital annotations placed on the femur to calculate leg length discrepancy. The system will make use of digital point annotations, preferably drawn by the user on reproducible points on the greater trochanter on both femurs, to calculate the difference in leg length between the operative and contralateral anatomy. The system may mathematically correct for differences in femoral alignment, relative to the pelvis, when calculating the leg length inequality by mathematically correcting for differences in femoral alignment. In a preferred implementation, this is performed by rotating the operative femur around the estimated center of rotation so that it matches the contralateral femur position relative to the pelvis.

The development of a system and method that can accomplish this technique relies upon customized software. One implementation of this system can be done by using an iPad mobile computing device. The software to develop this system on this device is written in the C# programming language using a Mono compiler. Development is performed using a MVC (model-view-controller) architecture which decouples the screen schematics and visualization from the software code that actually performs the analysis and other required calculations. Additionally, the data for this system can be stored locally on the device, or otherwise on a remote server, and is accessible and used by the C# software code when the system is being used.

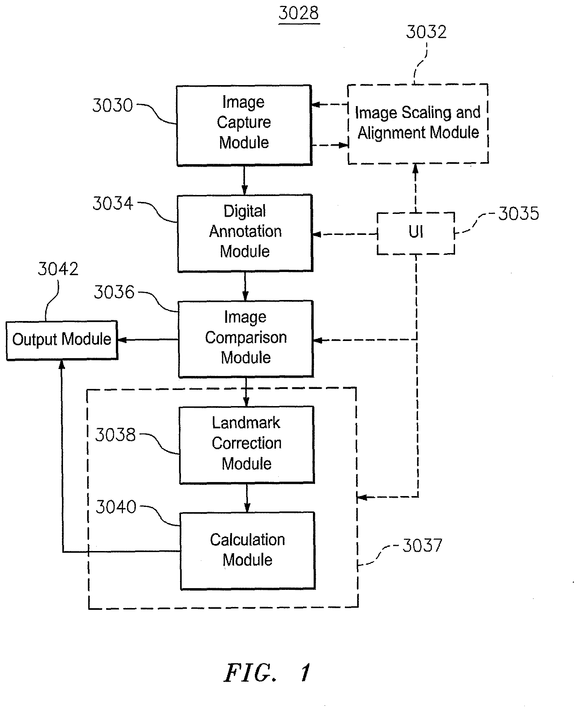

In a preferred construction, this method begins with an Image Capture Module 3030, FIG. 1, capturing a preoperative AP bilateral image of the pelvis in Step 3702, FIG. 2. This image may be captured in a variety of ways, such as being loaded from a PACS or other digital imaging system using DICOM or other communication protocol or else via a direct digital transmission with a radiographic imaging system using a wireless or physical network connection. Alternatively, an image may have been previously loaded into the system, and may be obtained from the computing device or server's storage system connected with the software system. Consistent with standard orthopaedic practice, a ball marker of known size may be included in the captured image to be used for scaling in the next method step.

After capturing the image, in one construction an optional Image Scaling and Alignment Module 3032, FIG. 1, scales the image in step 3704, FIG. 2, if the images have not been previously scaled. To scale the image, a ball marker is generally placed in the image. The module 3032 can use this ball marker to scale the image by automatically identifying the ball marker, using image recognition software code, and then recording the known size of the ball marker in order to apply absolute scaling to the image.

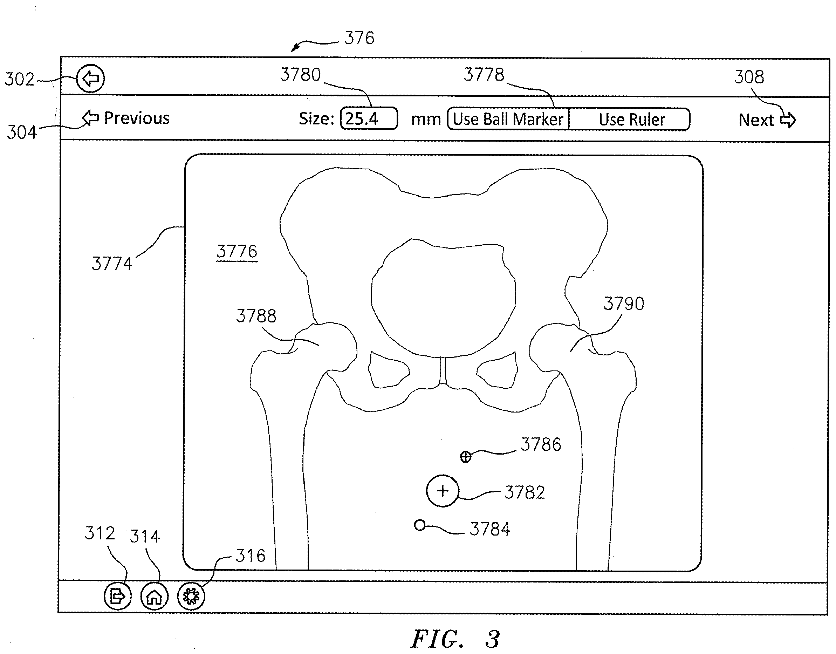

FIG. 3 is a schematic digital screen view of a software application running in a mobile computing device, such as an iPad, Android tablet, or Microsoft Surface tablet, showing the scaling in Step 3704, FIG. 2, of a preoperative image of the bilateral AP pelvis captured in Step 3702, FIG. 2. An image 3776 is contained within a digital image frame 3774, displaying the captured radiographic image of a preoperative bilateral AP pelvis. The ipsilateral preoperative hip/femur 3790 and contralateral hip/femur 3788 are visible in the captured image 3776. A ball marker, placed external to the patient body when the x-ray image was taken, has been automatically identified by the software's image recognition algorithms, and a digital circle annotation 3782 has been automatically placed by Image Scaling and Alignment Module 3032, FIG. 1, so that the digital circle overlays the physical ball marker in the image. A movement handle 3784 and a sizing handle 3786 enable the user to move and resize the digital circle 3782, so that it can be adjusted if there are any inaccuracies in the automated placement of the object in relation to the ball marker, or if the user wants to use a different object of known size to scale the image. Input box 3780 allows the user to specify the known size of the object used to scale the image, with a pre-set value of 25.4 mm, indicating the standard size for ball markers used in orthopaedic imaging. Toggle 3778 enables the user to choose whether they wish to use a ball marker or a linear 'ruler' object for scaling, should they choose an alternative linear object to scale the image. When the user is done with the scaling process, they will select the clickable 'Next' button 308 to continue to the next step in the process.

The method continues with an optional User Interface UI 3035, FIG. 1, guiding the placement of a series of digital annotations on the ipsilateral hip in Steps 3706 - 3712, FIG. 2, followed by annotation placement on the contralateral hip in Step 3714 - 3718, FIG. 2. Preferred constructions will make use of automated image recognition algorithms to identify ipsilateral bony anatomy, thereby automatically placing these digital annotations and relying upon the user to merely adjust them if they are not placed correctly. This automated image recognition may be used in any of the steps 3706 through 3718, FIG. 1, that guide the user through placement of digital annotations on bony anatomy.

In Step 3706, FIG. 2, the Digital Annotation Input Module 3752, FIG. 1, guides the placement of a digital point on the greater trochanter, an identifiable point on femoral anatomy, on the operative side. While alternative constructions of this system may use any identifiable point on the femur, the greater trochanter is an ideal point because the anatomy is less sensitive to rotation when using 2-d radiographic imaging compared to other identifiable points, such as the lesser trochanter. As described previously, preferred constructions of this system may make use of image recognition to automatically guide the point placement, and then the system will allow the user to modify the placement of the digital point.

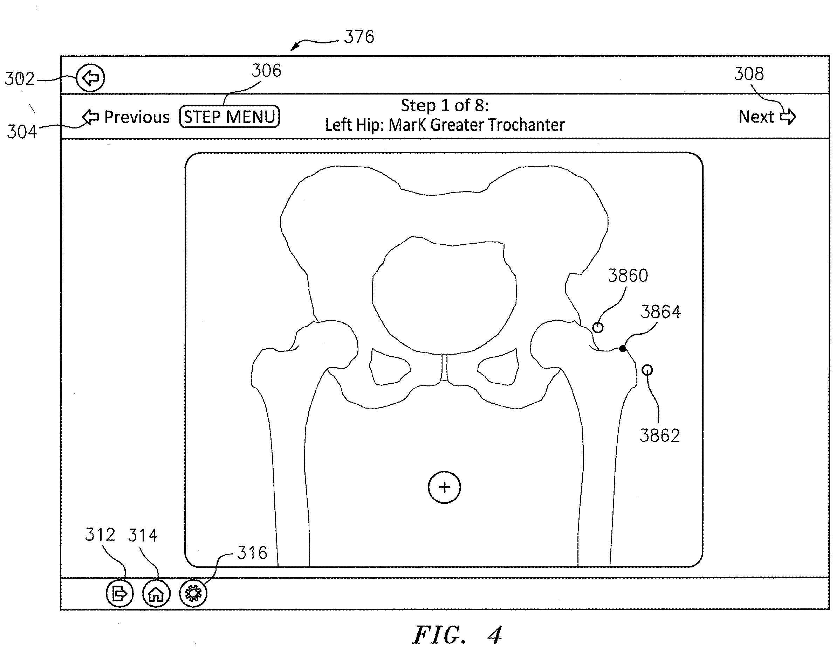

FIG. 4 is a schematic digital screen view of step 3706, FIG. 2, in which Digital Annotation Input Module 3752, FIG. 1 guides placement of a digital point 3864 on an identifiable spot on the greater trochanter on the operative hip. The digital point may initially be placed by image recognition, and then a user may manipulate the point position using movement handle 3860 or 3862. The Clickable "<Previous" button 304 allows the user to return and redo the previous step in the process, while the clickable "Next>" button 308 brings the user to step 3708, FIG. 2. The "Step Menu" button 306 provides the user with the ability to navigate backward to redo specific steps throughout the implementation of this system.

In Step 3708, FIG. 2, Digital Annotation Module 3034, FIG. 1, guides the drawing and positioning of a digital circle annotation so that it surrounds the femoral head of the ipsilateral hip. This annotation data will be used later in this system to estimate the center of rotation of the ipsilateral hip, which will be used later by Landmark Correction Module 3038, FIG. 1 to mathematically account for any differences in femoral alignment or abduction, relative to the pelvic anatomy, between the ipsilateral and contralateral hip. Landmark Correction Module 3038 and Calculation Module 3040 can be considered as components of an Analysis Module 3037, shown in dashed lines.

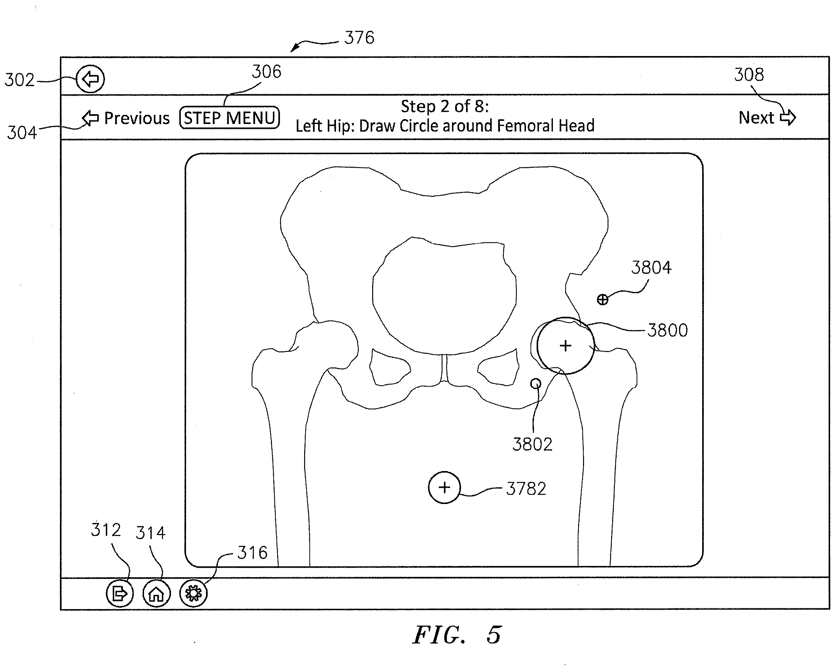

FIG. 5 is a schematic digital screen view of step 3708, FIG. 2 in which Digital Annotation Input Module 3752, FIG. 1 guides placement of a digital circle annotation 3800 around the ipsilateral femoral head. The circle may initially be placed by image recognition, and then a user may manipulate the size and position of the object by using movement handle 3802 and sizing handle 3804. The Clickable "<Previous" button 304 allows the user to return and redo the previous step in the process, while the clickable "Next>" button 308 brings the user to step 3710, FIG. 2.

In Step 3710, FIG. 2, Digital Annotation Module 3034, FIG. 1, guides the placement of an annotation that appears as the lower portion of a hip prosthetic, referred to as the "femoral axis tool', to identify the longitudinal axis of the ipsilateral hip. In a preferred implementation, the annotation used is a digital rendering of a hip template with the neck and head of the prosthetic removed. The user can reposition the annotation so that it fully fills the femoral canal, enabling the system to identify the location of the longitudinal axis of the femoral canal. This digital rendering annotation contains a line that continues along the longitudinal axis of the annotation, so that the location of the midpoint of the femur can be clearly identified in the image.

Alternative constructions may use other annotations to identify the midpoint of the femoral canal. For example, the user may be prompted in alternative constructions to draw a digital line down the central axis of the femur, or else a rectangular object that can be positioned along the femoral cortices so that the central axis of the femur can be calculated mathematically. However, the preferred construction makes use of an annotation that looks like the distal portion of a hip prosthetic because it has been found to be more accurate in correctly determining the longitudinal axis of the femur, whereas alternative constructions have been found to have more error due to the widening on the proximal femur.

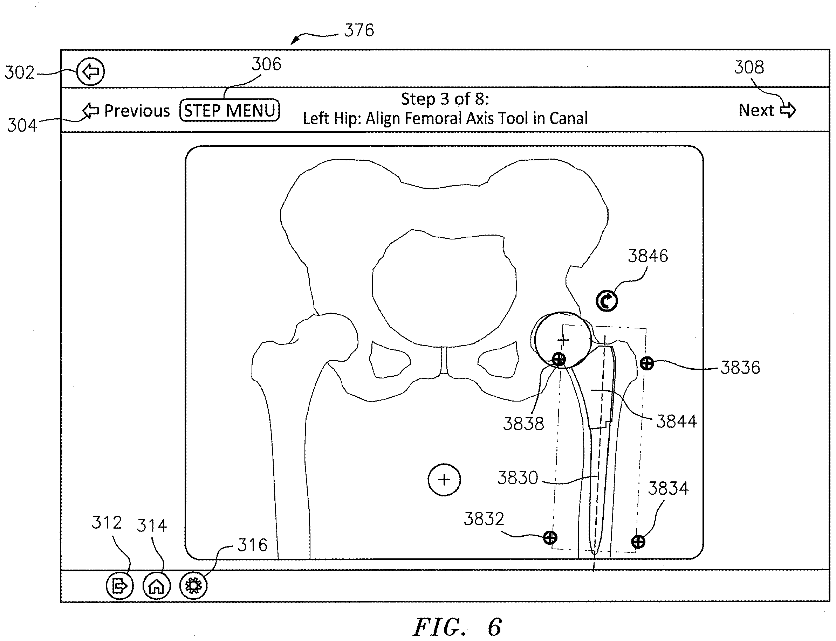

FIG. 6 is a schematic digital screen view of step 3710, FIG. 2, in which digital femoral axis tool 3844, containing digital line 3830 that identifies the longitudinal axis of the femur, is positioned within the femoral canal. In alternative constructions, a rectangular object or other structure may be used to identify the longitudinal axis of the femur. In preferred implementations of this system, Digital Annotation Module 3034, FIG. 1, will initially attempt to auto-position the femoral axis tool in the femoral canal using a combination of image recognition software routines and known position data such as the previously identified position of the greater trochanter. Movement and sizing handles 3832, 3834, 3836, and 3838 enable the user to move and resize the femoral axis tool, and rotation handle 3846 enables the user to rotate the femoral axis tool so that it is optimally positioned within the femoral canal. Clickable button "<Previous" 304 will allow the user to repeat step 3708, FIG. 2, and clickable button "Next>" 308 brings the user to step 3712, FIG. 2.

In Step 3712, FIG. 1, Digital Annotation Module 3034, FIG. 1, guides the placement of a digital line that connects two identifiable point on the pelvic anatomy. For example, the line may be drawn to connect a point on the inferior pubic symphysis to the anterior superior iliac spine. The line may alternatively be drawn between other readily observable points on the pelvis, such as the teardrop, superior pubic symphysis, etc. In a later step, a similar digital line will be drawn to connect the same anatomic points on the pelvic anatomy on the contralateral side of the image, and the system will then use these lines to align and overlay the ipsilateral and contralateral anatomy.

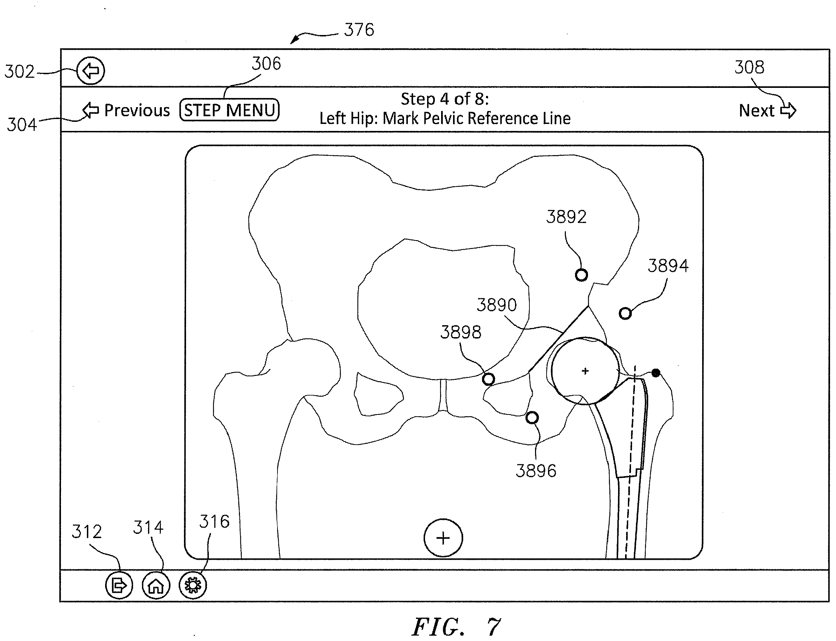

FIG. 7 is a schematic digital screen view of step 3712, FIG. 1, in which Digital Annotation Module 3034, FIG. 1, guides placement of a digital line annotation 3890, FIG. 7, connecting reproducible points on the ipsilateral side of the pelvic anatomy. In this case, pelvic reference line 3890 connects a point on the pelvic teardrop to an identifiable pelvic point on the anterior superior iliac spine. Preferably, Digital Annotation Module 3034, FIG. 1 will initially position the digital line using the system's image recognition combined with known anatomic point data. The user may manipulate precise positioning of pelvic line 3890 using navigation handles 3892, 3894, 3896, and 3898, which are used to position the digital line end points. The Clickable "<Previous" button 304 allows the user to return and redo the previous step in the process, while the clickable "Next>" button 308 brings the user to step 3714, FIG. 2.

The method continues in Step 3714, FIG. 2 with Digital Annotation Module 3034, FIG. 1 guiding the placement of a digital point annotation on the contralateral greater trochanter, similar to the annotation placed on ipsilateral anatomy in Step 3706, FIG. 2.

In Step 3716, FIG. 2, Digital Annotation Module 3034, FIG. 1 guides identification of the longitudinal axis of the contralateral hip. Note that the specific construction of this step will match what was done in step 3710 on the operative side, but within the contralateral anatomy. If a femoral axis tool annotation was positioned down the midline of the femur in step 3708, a similar construction will be used in this step. Alternatively, if a rectangular digital annotation of stem prosthetic annotation was used to identify the axis by aligning with the medial and lateral femoral cortices in step 3710, a similar construction will be used in this step.

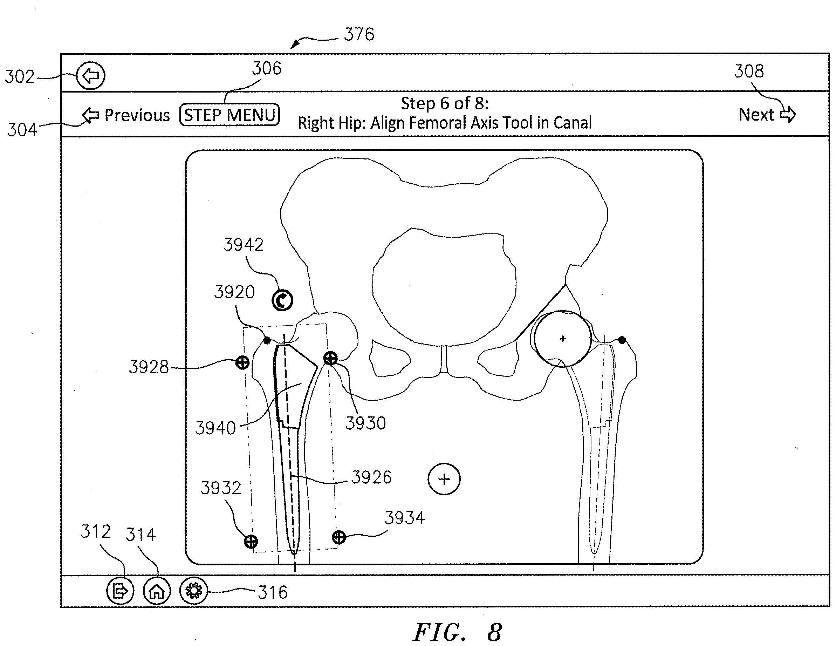

FIG. 8 is a schematic digital screen view showing Step 3716, FIG. 2, with Digital Annotation Module 3034, FIG. 1, guiding placement of a digital femoral axis tool 3940 containing digital line 3926 that identifies the longitudinal axis of the femur, along the femoral canal of the contralateral femur. Digital point 3920 that appears on the contralateral greater trochanter, was previously placed in Step 3714, FIG. 2 by Digital Annotation Module 3034, FIG. 1, and still appears in this step. Preferred constructions will use image recognition to initially position both femoral axis tool 3940 and point 3920. The user may manipulate precise positioning of femoral axis tool 3940 using navigation handles 3928, 3930, 3932, and 3934, along with rotation navigation control 3942 that enables a user to rotate the digital annotation. Note that FIG. 8 shows that the system guides the user in closely matching contralateral annotations as compared to what was annotated on the ipsilateral anatomy. The Clickable "<Previous" button 304 allows the user to return and redo the previous step in the process, while the clickable "Next>" button 308 brings the user to step 3718, FIG. 1.

In Step 3718, FIG. 2, Digital Annotation Module 3034, FIG. 1, guides the placement of a digital line on the contralateral pelvis. The pelvic line drawn should connect the same anatomic points on the contralateral side of the captured image as the pelvic line that was drawn on the operative side in Step 3712, FIG. 2.

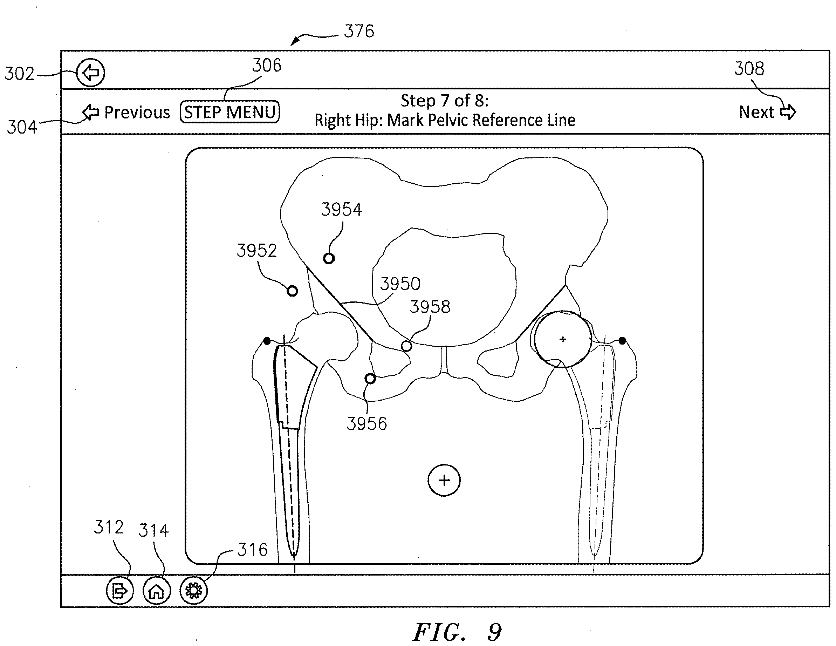

FIG. 9 is a schematic digital screen view showing Digital Annotation Module 3034, FIG. 2, guiding placement of a digital line annotation 3950 on the contralateral pelvic anatomy in Step 3718, FIG. 2. The endpoints of digital line 3950 are consistent with the pelvic line endpoints drawn on the ipsilateral anatomy, which in this construction are points on the pelvic teardrop and identifiable anatomy on the anterior superior iliac spine. Navigation handles 3952, 3954, 3956, and 3958 enable the user to modify the position of digital line 3950. The Clickable "<Previous" button 304 allows the user to return and redo the previous step in the process, while the clickable "Next>" button 308 brings the user to step 3720, FIG. 2.

In Step 3720, Fig. 1, Image Comparison Module 3036, FIG. 1, will take the annotated image of the AP pelvis and separate it into two images, one of the contralateral side and one of the operative side. In various constructions, the module may use slightly different techniques and algorithms to separate the initially captured image and create two images from it. In a preferred construction, the module will separate the image into a contralateral and operative image by transecting the two pelvic reference lines that were drawn in steps 3712 and 3718, FIG. 2. Effectively, this splits the images along the center of the pubic symphysis. Preferably, the system will then increase the medial viewing area of each image in the separated images, so that there is some overlap and better visualization of the images available once they are fully separated. With respect to the lateral border of each image, the entire lateral side of the images can be retained in the separated images. Alternatively, the identification of the greater trochanter and femoral axis line can be analyzed by the processing module to identify where the lateral side of each image can be truncated.

In some alternative constructions, the original image of the AP pelvis may be simply retained and copied to create a second image, with the second image inverted along its horizontal axis, so that the ipsilateral and contralateral images may be overlaid according to pelvic anatomy. In this construction, the pelvic reference lines annotated on the ipsilateral and contralateral anatomy can be used in Step 3722, FIG. 2, to align the two images so that they can be used to to analyze leg length discrepancy.

In Step 3722, FIG. 1, Image Comparison Module 3036, FIG. 1, overlays the ipsilateral hip image and flipped contralateral hip image by using the pelvic reference lines previously drawn. In a preferred implementation, the images are positioned by the Image Comparison Module by overlaying the inferior pelvic points identified on the pelvic overlay lines in the separated images. The Module can then rotate the two images, relative to one another, so that the pelvic lines are along the same axis.

In preferred implementations of this system, the Image Comparison Module 3036, FIG. 1, will rotate but NOT scale, the two images relative to one another in Step 3722, FIG. 1. Therefore, the overlay lines may be of different length on the superior portion of the line. This is appropriate due to various inaccuracies in trying to scale images based on ipsilateral and contralateral anatomy. These sources of inaccuracy include pelvic asymmetry, potential pelvic obliquity (particularly due to patient mal-positioning due to preoperative pain), and potentially parallax. Nevertheless, certain situations and surgical preferences dictate that these may be some deviation in this area, and specific surgeons will have situations when they would like to scale the images with respect to the overlay lines. To accomplish this, various configuration options may be available in the software to allow various modifications to the processing system, such as modification of the method to apply scaling to the separated images.

In Step 3724, the Landmark Correction Module 3038, FIG. 1, corrects for differences in contralateral and operative femoral positioning when calculating the leg length discrepancy. This can be achieved by mathematically rotating the longitudinal axis of the ipsilateral femur to align it with the longitudinal axis of the contralateral femur. This is done by rotating the identified point on the greater trochanter around the femoral center of rotation. In certain constructions, an independent femoral abduction analysis is conducted.

In Step 3726, Calculation Module 3040, FIG. 1 calculates the leg length inequality. In one implementation, the system accomplishes this by using the overlaid images to calculate the distance, along the axis of the femur as identified by the longitudinal axis line, between the ipsilateral and contralateral greater trochanter points. The system uses a point that includes correction for any differences in femoral abduction when calculating this distance. Coinciding with the mathematical calculation in this Step 3726, FIG. 1, the Output Module 3042, FIG. 1, displays the output rendering of the two overlaid images, along with the calculated leg length discrepancy that has accounted for differences in femoral positioning or abduction, relative to the pelvis, between the contralateral and ipsilateral images.

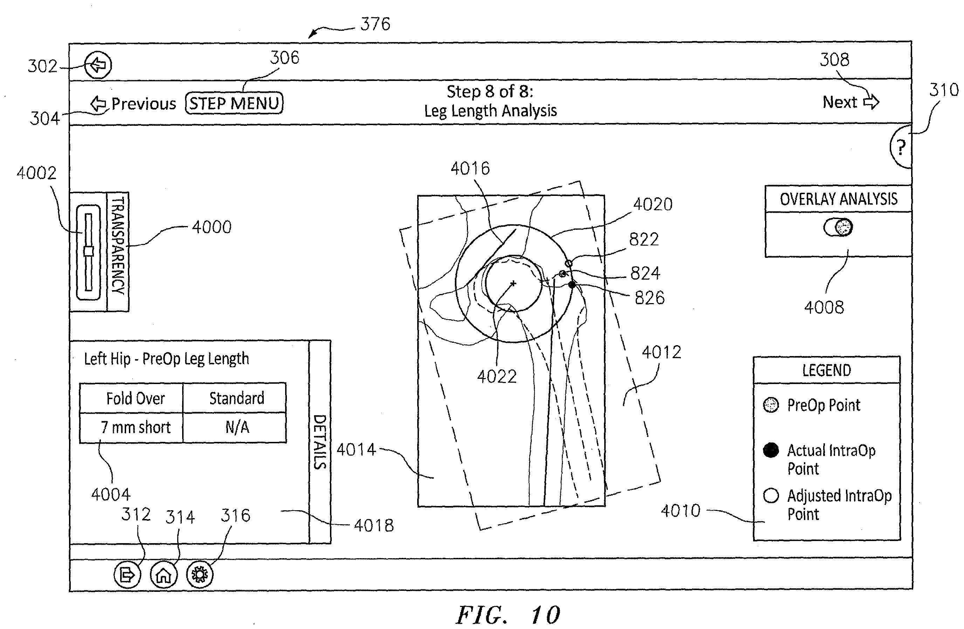

FIG. 10 is a schematic digital screen rendered by Output Module 3042, FIG. 1, showing one rendering of the separated contralateral and ipsilateral images overlaid on one another, along with the leg length discrepancy calculation from Step 3726, FIG. 2. Digital image frame 4012 contains an overlay of the ipsilateral hip, along with the inverted contralateral hip in digital frame 4014. Digital line 4016 shows how Image Comparison Module 3036, FIG. 1 has automatically overlaid the ipsilateral and contralateral images based on pelvic anatomy, as described in the method. The calculated leg length discrepancy, identified by reference 4004, indicates a shorter ipsilateral length of 7mm is displayed within digital frame 4018. Legend 4010 provides a legend that shows the identified greater trochanter points on the ipsilateral anatomy and contralateral anatomy, as well as the point that has been adjusted to account for femoral abduction which is used for mathematical analysis of leg length. The correction for the difference in femoral positioning is calculated using the circle 4020, through which the greater trochanter points can be rotated around the center of rotation 4022. Digital annotation points 824 and 826 indicate the positioning of the points positioned on the femoral anatomy, and digital annotation point 822 has been generated by the system to correct for femoral abduction and is used to determine the leg length differential identified by reference 4004. The Toggle icon 4008 enables the user to show the femoral abduction correction point or otherwise turn it off so that the overlaid images are simply displayed. Transparency slider control 4002, contained within digital frame 4000, allows the user to adjust the transparency of the overlaid images 4014 and 4016. The clickable command button labelled "<Previous", 304, allows the user to return to an earlier step, and the clickable "Next" button 308 completes the process and allows the user to complete and exit the system.

Alternative constructions will change the order of various steps without materially altering the system and method. In particular, the order of annotation registration on the anatomy is inconsequential to the system and method beyond preferred methods of usability. Alternative constructions may guide the user to identify and annotate the same anatomic points consecutively on the ipsilateral and contralateral sides (i.e. the greater trochanter may be registered first on the ipsilateral side, and in the following step the greater trochanter can be registered on the contralateral side.) In another alternative construction, all points on the contralateral side may be identified, followed by registration of the operative hip.

Additional alternative construction may change the hip that is flipped so that the operative, rather than the contralateral, hip is flipped. While this may be confusing to the user that is focused on analysing the operative hip and affect the overall usability of the system, the results and ability to interpret the information would not materially change in this construction.

Similarly, alternative constructions may separate the AP pelvis image into separate contralateral and operative images earlier than the construction presented. For example, the pelvic lines drawn in Steps 3712 and 3718, FIG. 2, may be the initial annotations drawn in an alternative construction, after which the image could be separated, with additional annotations performed after the separation of the original image into images of the contralateral and ipsilateral hip.

Alternative constructions may handle correction of femoral positioning differently. An alternative construction will guide the user to estimate the center of rotation of the contralateral hip by drawing a circular annotation around the femoral head rather than the ipsilateral hip. While this may confuse the user from a usability perspective, because they are interested in analysing the operative hip, the mathematical and visual analysis will be consistent.

In yet another construction, and based on differences between surgeons in surgical methodology and philosophy, the user may elect to entirely ignore any differences in femoral positioning. In this construction, the step to identify the center of rotation may either be ignored by the software when calculating leg length discrepancy or, alternatively, may be entirely removed from the method. A preferred way to implement these different constructions is by providing various software configuration options in the software that can slightly change the steps and/or processing according to the surgeon's preference.

Alternative constructions may use different annotation in various annotation registration steps to accomplish the same objective. For example, a much smaller point than what has been illustrated may be used to identify the greater trochanter, and/or the annotation may be modified to show cross hairs that can help the user in their precise registration of particular anatomy. Transparency of these annotation objects may also change in various constructions. Some users may prefer a solid object, while others will prefer a partially transparent object that enables the user to visualize the radiographic image underlying each respective annotation. As described previously, the digital line used to identify the longitudinal axis of the respective femurs may be replaced with a rectangular object than aligns with the femoral cortices, or a template-like object that does the same, or other such object that helps in precise identification of the axis.

The implementation of this system described above enables the user to more accurately analyse preoperative differences in leg length more effectively by accounting for femoral abduction. An additional preferred implementation of the system, which also corrects for femoral abduction differences, can be used to guide the surgeon intraoperatively when analysing at least one of leg length and offset.

The operation of one intraoperative analysis system according to the present invention is depicted in flowchart OA in FIG. 11, beginning by capturing, Step 4700, either a selected preoperative ipsilateral image, or a selected inverted contralateral image. Whichever image is selected is referred to herein as a "first, reference image" or "preop image".

In this construction, the Image Capture Module 3030, also referred to as an Image Selection Module, of overlay analysis system 3028, FIG. 1 captures an image of the preop hip. Image capture can be implemented in a variety of ways, such as a direct connection to a c-arm fluoroscopy unit or other radiographic imaging system, file upload, or other techniques such as accessing the radiographic image over a network using a DICOM or similar communication protocol. Implementations that operate on a mobile device such as an iPad, or other platforms that similarly integrate a camera device, may alternatively capture the preop image by prompting the user to take a picture of the images using the device camera. If an inverted contralateral image is used as a 'preop' image, the contralateral image may be captured and then inverted within the software. Alternatively, it may be inverted in another system, such as a fluoroscopic c-arm like the GE OEC Elite 990, before being loaded into this system by the Image Capture Module 3030.

After capturing the preop image in Step 4700, FIG. 11, Digital Annotation Module 3034, FIG. 1 guides the placement of various digital annotations on the preop image in Steps 4702 - 4706, FIG. 11.

In Step 4702, FIG. 11, Digital Annotation Module 3034, FIG. 1 guides the placement of a digital point annotation on the greater trochanter within the preop image. As described in the previous embodiment, the greater trochanter is a particularly useful femoral landmark to annotate because it is easily identifiable and because the anatomy is relatively insensitive to deviations in image acquisition. A digital annotation point may be placed on the lesser trochanter or another identifiable femoral landmark in alternative system constructions. However, consistent point placement on the lesser trochanter is more susceptible to error originating from deviations in image acquisition angle based on its 3-dimensional anatomy. In a preferred implementation, the system will attempt to automatically identify the greater trochanter using image recognition and then enable the user to modify the digital point placement.

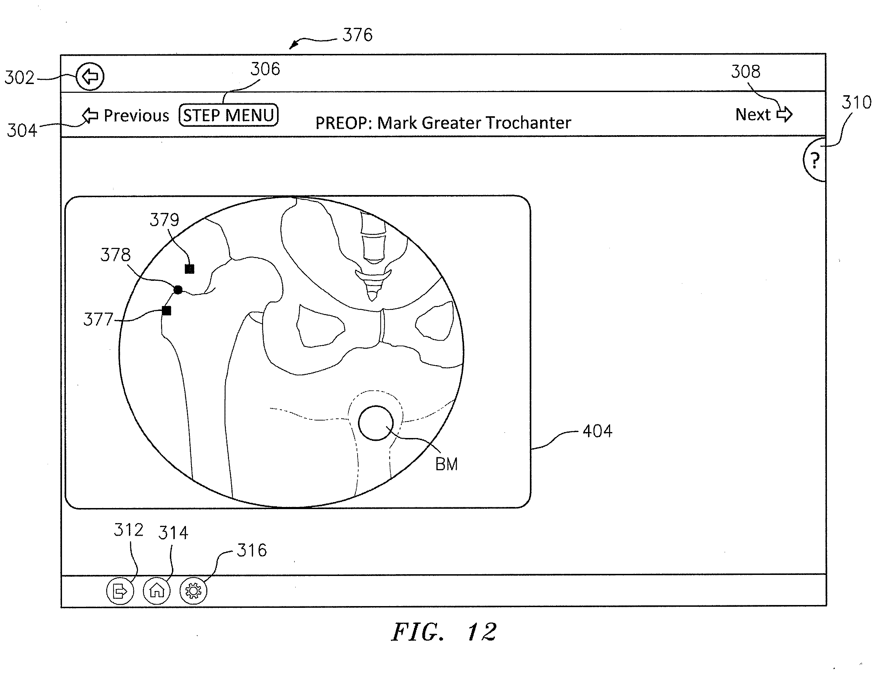

FIG. 12 is a schematic digital screen view 376 of Step 4702, FIG. 11, with a captured preop image of the right side of a patient's preoperative hip shown within digital frame 404. Digital Annotation Module 3034, FIG. 1 guides placement of digital point annotation 378 on an identifiable position on the greater trochanter within this preop image. Digital movement handles 377 or 379 enable the user to position the digital point annotation 378 on touch-screen devices, such as an iPad, without the user's fingers obscuring the positioning of the annotation 378. Clickable icon button 302 allows the user to exit the system at any point, and the "<Previous" button 304 allows the user to return and redo the previous step in the process, while the clickable "Next>" button 308 brings the user to the next step in the method. Clickable help icon 310 will open an on-screen guide that explain system functionality.

In Step 4704, FIG. 11, Digital Annotation Module 3034, FIG. 1, guides the placement of an annotation that appears as the lower portion of a hip prosthetic, referred to as the 'femoral axis tool', to identify the longitudinal axis of the preop hip image. In a preferred implementation, the annotation used is a digital rendering of a hip template with the neck and head of the prosthetic removed. The user can reposition the annotation so that it fully fills the femoral canal, enabling the system to identify the location of the longitudinal axis of the femoral canal. This digital rendering annotation contains a line that continues along the longitudinal axis of the annotation, so that the location of the midpoint of the femur can be clearly identified in the image. As with the previous embodiment, this femoral axis tool can be substituted with the use of a digital line, rectangular annotation, or other such annotation that can be used to identify the longitudinal axis of the femur.

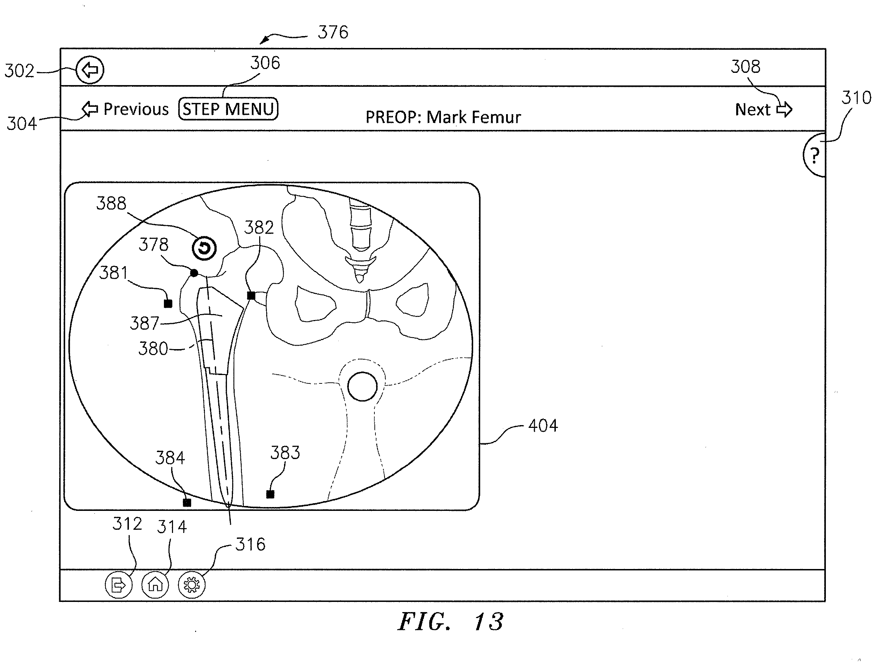

FIG. 13 is a schematic digital screen view of 376 showing Step 4704, FIG. 11, in which digital femoral axis tool 387, containing digital line 380 that identifies the longitudinal axis of the femur, is positioned within the femoral canal in the preop image 404. (In alternative constructions, a rectangular object or other structure may be used to identify the longitudinal axis of the femur.) In preferred implementations of this system, Digital Annotation Module 3034, FIG. 1 will initially attempt to auto-position the femoral axis tool in the femoral canal using a combination of image recognition software routines and known position data such as the previously identified position of the greater trochanter. Movement and sizing handles 381, 382, 383, and 384 enable the user to move and resize the femoral axis tool, and rotation handle 388 enables the user to rotate the femoral axis tool so that it is optimally positioned within the femoral canal. Digital point annotation 378, placed on the greater trochanter in a previous step, is visible on screen. Various control icons guide navigation and additional features within the system. Clickable button 302 allows the user to exit the system at any time and terminate the process. Clickable "<Previous" button 304 will allow the user to return to the previous step, and clickable button "Next>" button 308 brings the user to the following step in the system. Help icon 310 will activate a digital help guide upon user selection that guides the user in system use. Icon 312 allows the user to log out and fully exit the system, and selecting icon 314 will return the user to the start of the process. Configuration icon 316 will open a configuration screen with the opportunity to change the preferred implementation of the system. For example, a configuration option in some implementations may include the ability for a user to select a different geometric shape or alternative digital rendering of a portion of a prosthetic to aid in identifying the longitudinal axis of the femur.

In Step 4706, FIG. 11, Digital Annotation Module 3034, FIG. 1, guides the placement of a digital pelvic reference line that connects two identifiable points on the pelvic anatomy in the preop image. In one implementation of this step, the line will be drawn to connect a point on the inferior pubic symphysis to an identifiable point on the anterior superior iliac spine. In a later step, a similar digital line will be drawn to connect the same anatomic points on the pelvic anatomy in the intraoperative image. The system will then use these lines to align, apply scaling, and overlay the preop and intraop images.

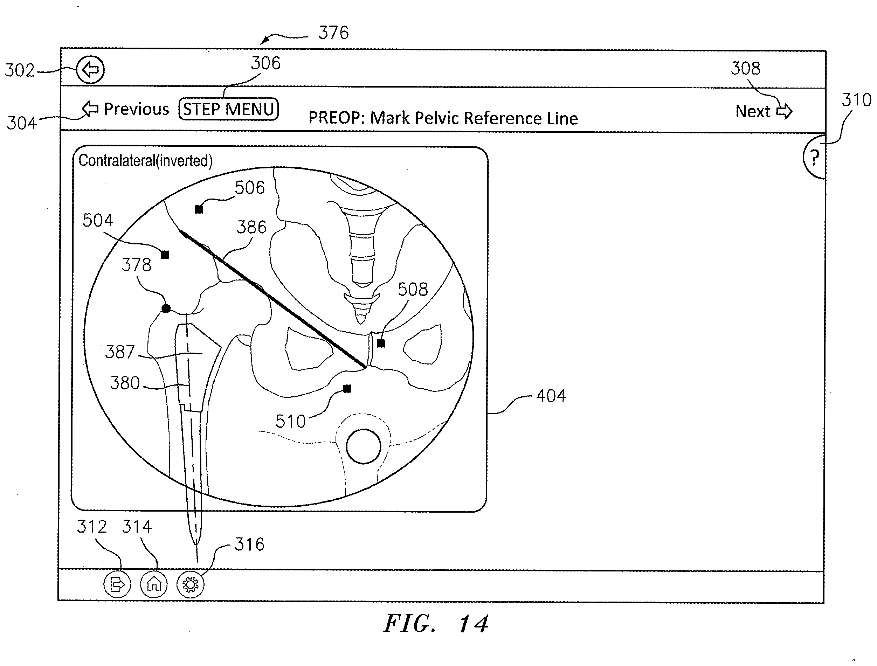

FIG. 14 is a schematic digital screen view 376 of Step 4706, FIG. 1, in which Digital Annotation Module 3034, FIG. 1, guides placement of a digital line annotation 386, FIG. 11, connecting reproducible points on the preoperative pelvic anatomy within captured preop image 400. In this screen view, pelvic reference line 386 connects the inferior point on the pubic symphysis to an identifiable pelvic point on the anterior superior iliac spine. Preferably, Digital Annotation Module 3034, FIG. 1 will initially position the digital line using the system's image recognition combined with known anatomic point data gathered in previous steps. The user may manipulate precise positioning of pelvic reference line 386 using navigation handles 504, 506, 508, and 510, which are used to position the digital line end points.

Once the surgeon has implanted a trial hip prosthetic and associated acetabular component prosthetic during the surgical procedure, the method can continue by capturing and digitally annotating an intraoperative image.

In Step 4708, FIG. 11, Image Capture Module 3030, FIG. 1 guides the capture of an intraoperative image. Generally, an intraoperative image will be taken using a fluoroscopic c-arm, such as the GE OEC Elite 990 fluoroscopic c-arm, or other digital radiography system, and the image may be captured using one of the methods described previously.

In Step 4710, FIG. 11, Digital Annotation Module 3034, FIG. 1 guides the placement of a digital point annotation on the greater trochanter (or other point on the femoral anatomy) within the intraop image in a similar manner to how it was done in the preop image in Step 4702, FIG. 11. It is critical that the digital annotation is placed on the same anatomic point on the femur in both the preop and the intraop images.

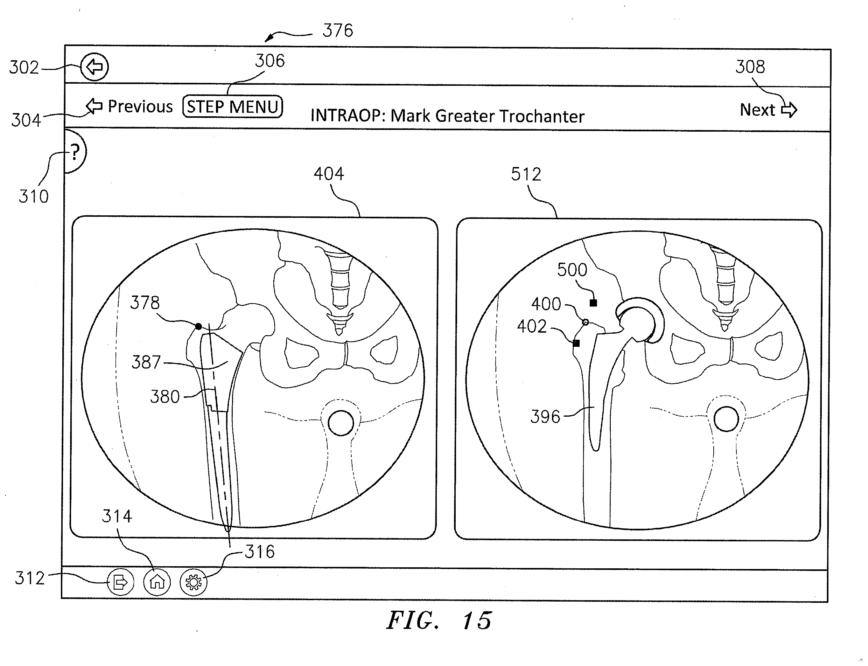

FIG. 15 is a schematic digital screen view 376 of Step 4710, FIG. 11, with a captured preop image 404 of the right side of a patient's preoperative hip and an intraop image 512 of the right side of the patient's intraoperative hip. Digital Annotation Module 3034, FIG. 1 guides placement of digital point annotation 400 on an identifiable position on the greater trochanter within intraop image 512. Digital movement handles 402 and 500 enable the user to position the digital point annotation 400 on touch-screen devices, such as an iPad, without the user's fingers obscuring the positioning of the annotation 378. Within the digital screen view 376, femoral axis tool 387 with longitudinal axis 380, and digital point annotation 378 on the greater trochanter, remain visible as digital annotations within preop image 404.

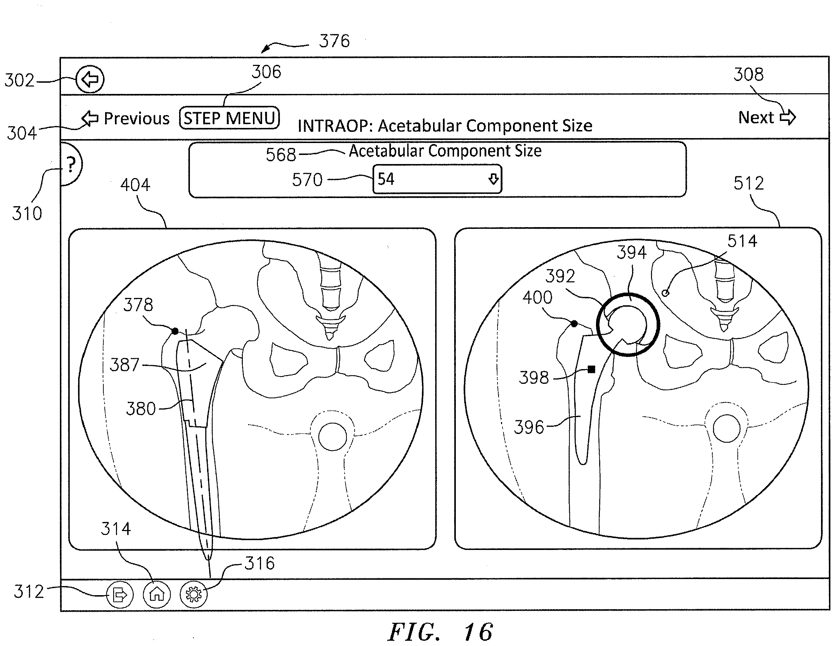

In Step 4712, FIG. 11, the Digital Annotation Module 3034, FIG. 1, guides the positioning of a digital circle annotation so that it overlays the boundary of the acetabular component, as shown by digital circle 392 in FIG. 16. Positioning this digital circle annotation can additionally be used to identify the center of the digital circle annotation, which corresponds to the center of rotation of the intraop hip. This information which will be used in a later step to assist in correcting for differences in femoral abduction between the preop and intraop images. Additionally, the user can enter the known size of the acetabular component, via User Interface UI, box 3035, which may be used to scale the images in a later step.

In a preferred implementation of this system, Digital Annotation Module 3034, FIG. 1 will auto-detect the location of the implanted acetabular component in the intraop image using image recognition algorithms, and the system will then generate a digital circle annotation. The system will then allow the user, via User Interface UI, box 3035, to adjust the size and position of the digital circle. In alternative constructions, the user estimates the approximate center of rotation by drawing or positioning a circle around the femoral head in the preoperative image, and utilizing the center of that circle as an estimate of the center of rotation.

FIG. 16 is a schematic digital screen view 376 of Step 4712, FIG. 11, in which Digital Annotation Module 3034, FIG. 1 has auto-positioned a digital circle annotation 392 in intraop image 512 around the acetabular component 394 that has been surgically implanted. Femoral trial prosthetic 396, implanted during surgery, is also identifiable within intraop image 512. The digital circle may be either generated using image recognition to identify the acetabular component, positioned by the user, or initially system-generated in an approximate location and then positioned by the user. Movement handle 398 and sizing handle 514 allow the user to modify the position and size of the circle annotation 392. The size of the acetabular component is known because the surgeon has implanted it into the patient's anatomy. The known size of the component, "54 mm", is entered into digital drop down control 570 under digital text label "Acetabular Component Size" 568 positioned near the top of the screen. The digital annotation 400 previously placed on the greater troch in intraop image 512 is also still visible in this screen. Digital femoral axis tool 387, with longitudinal axis 380, and digital point 378 on the greater trochanter, also remains visible within the preop image 404.

In Step 4714, FIG. 11, Digital Annotation Module 3034, FIG. 1, guides identification of the longitudinal axis of the intraop hip. In preferred implementations of this system, a digital femoral axis tool annotation will be positioned within the femoral canal similarly to how it was placed in the preop image. Alternatively, if a rectangular digital annotation of stem prosthetic annotation was used to identify the axis by aligning with the medial and lateral femoral cortices in step 4704, a similar annotation will be used in this step.

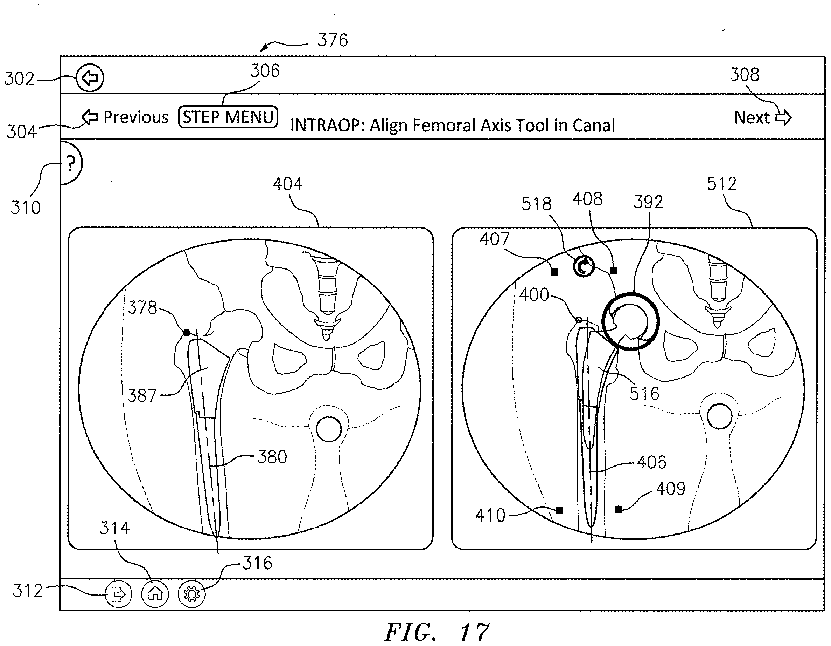

FIG. 17 is a schematic digital screen view 376 of step 4714, FIG. 11, in which digital femoral axis tool 516, containing digital line 406 that identifies the longitudinal axis of the femur is positioned within the femoral canal within intraop image 512. In preferred implementations of this system, Digital Annotation Module 3034, FIG. 1 will initially attempt to auto-position the femoral axis tool in the femoral canal using a combination of image recognition software routines and known position data such as the previously identified position of the greater trochanter. Movement and sizing handles 407, 408, 409, and 410 enable the user to move and resize the femoral axis tool, and rotation handle 518 enables the user to rotate the femoral axis tool so that it is optimally positioned within the femoral canal. Digital point annotation 400 in intraop image 512, positioned in a previous step, remains visible within this screen.

In Step 4716, FIG. 11, Digital Annotation Module 3034, FIG. 1, guides the placement of a digital pelvic reference line that connects two identifiable points on the pelvic anatomy in the intraop image. This digital line should be positioned between the same identifiable anatomic points on the pelvic anatomy in the intraop image as the digital line that was positioned on the preop image in Step 4706, FIG. 11. These pelvic reference lines will be used to align, scale, and overlay the preop and intraop images in a later step.

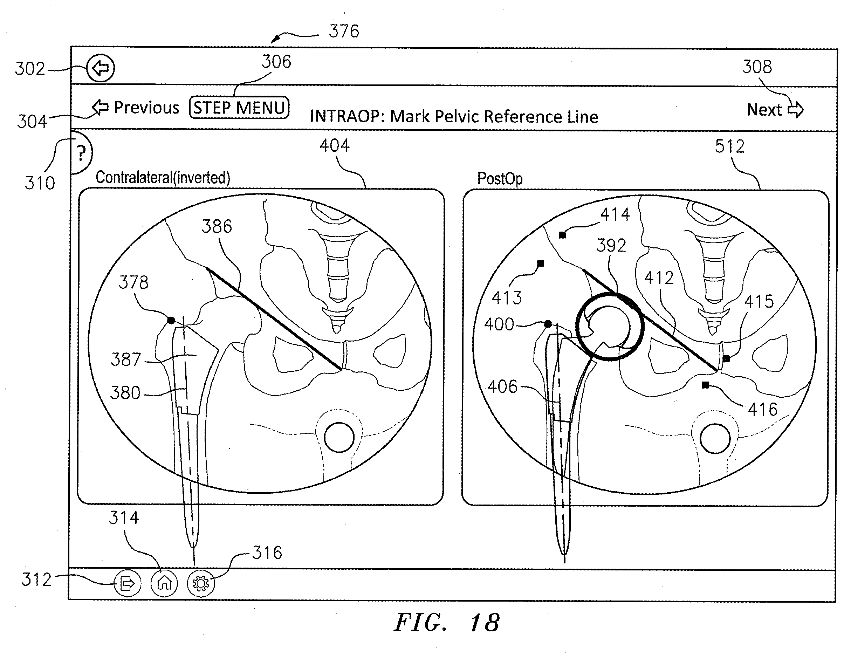

FIG. 18 is a schematic digital screen view 376 showing Digital Annotation Module 3034, FIG. 1, guiding placement of a digital line annotation 412 between two identifiable pelvic anatomic points within intraop image 512 in Step 4716, FIG. 11. The endpoints of digital line 412 are consistent with the pelvic line annotation 386 that was previously positioned in preop image 404, which in this example are points on the inferior pubic symphysis and identifiable anatomy on the anterior superior iliac spine. Navigation handles 413, 414, 415, and 416 enable the user to modify the position of digital line 412.

In Step 4718, FIG. 11, the Image Scaling and Alignment Module 3032, FIG. 1 scales and aligns the preop and intraop images using annotation data on the respective images. To scale the intraop image, the Image Scaling and Alignment Module 3032, FIG. 1 records the diameter in pixels of the circular annotation placed around the acetabular component in Step 4712, FIG. 11. The Module additionally receives the known diameter of the acetabular component in absolute (millimetre) distance. To determine the scale of the intraop image in absolute terms, the Module then divides the diameter of the acetabular component in millimetres by the diameter, in pixels, of the circular annotation surrounding the acetabular component. To scale the preop image, the Module next compares the distance, in pixels, of the pelvic reference line annotations drawn in Steps 4706 and 4716, FIG. 1, in the preop and intraop images, respectively. The Module changes the size of the preop image so that the size of the pelvic reference lines connecting similar anatomy in each image is the same. Because both images are similarly scaled according to consistent anatomic points identified on the pelvis, the absolute scaling ratio applied to the intraop image can also be applied to the preop image. Finally, the system rotates the preop image so that the pelvic reference lines in the preop and intraop images are now in the same orientation relative to the computing system's user interface. This alignment update ensures that the pelvis in the preop and intraop images are aligned, which will facilitate both correction for femoral abduction and overlaying of the images in later steps.

Image scaling of both the preop and intraop images may be performed in alternative constructions or configurations using alternative techniques. For example, a software system residing on a digital fluoroscopy system may have been used to align and scale the images prior to image acquisition by this system. Alternatively, the images may already be scaled and aligned because the surgeon took images with the patient and radiographic system in identical position with a known magnification ratio. The preop and intraop images may alternatively be scaled independently of one another using objects of known size in each image. For example, scaling can be applied independently in the preop image by drawing a digital line across diameter of the femoral head in the preop image, and entering the size in absolute terms. This absolute measurement is known during surgery because the surgeon traditionally extracts the femoral head and measures its size, using calipers, during hip arthroplasty. If there is prior hardware of known size previously inserted in the body of known size, this information could also be used to directly scale the images in alternative implementations of this system.

Image alignment in this step can also be accomplished in this step using alternative techniques. If the surgeon uses a table that secures the patient in an anterior position, such as the Hana table that is often used for hip surgery, and takes preop and intraop images using a fluoroscopic c-arm that doesn't move, image alignment may be preferred but not be absolutely required. In an alternative construction, the Module will guide the user to manually align the two images using an image overlay, or similar step, so that the user can manually align pelvic anatomy in the two images.

In Step 4720, the User Interface UI 3035, FIG. 1 asks the system if they want to include error analysis in the system. If Yes, then in Step 4722, FIG. 11, Digital Annotation Module 4722 will guide the positioning of one or more points on the pelvic anatomy for use in error analysis.

In Step 4720, FIG. 11, the Digital Annotation Module 3034, FIG. 1 asks via User Interface UI 3035, whether the user wants to include error analysis in the system output. If yes, Module 3034 prompts the user, in Step 4722, FIG. 11, to identify a set of anatomic points on the stationary pelvis in both the preop and intraop images. While a minimum of only one point is required to provide error analysis in the system, the system may guide the positioning of multiple points on the pelvic anatomy to analyse potential error. Sources of error in the system that may be identified by these points include drawing of the pelvic reference lines which can affect the image overlay, differences in imaging angle at the time of radiographic image capture, and differences in contralateral anatomy when the preop image used is actually an image of the contralateral hip. When the image overlay is created in a later step, these error analysis points can be visually inspected to examine the quality of the image overlay, so that any problems in the system may be visually identified.

The points selected for error analysis are ideally independent of the points used to create the pelvic reference lines in Steps 4706 and 4716 above. Ideal points will also be identifiable, such as a discernible point on the pelvic teardrop, ischial tuberosity and pubic symphysis.

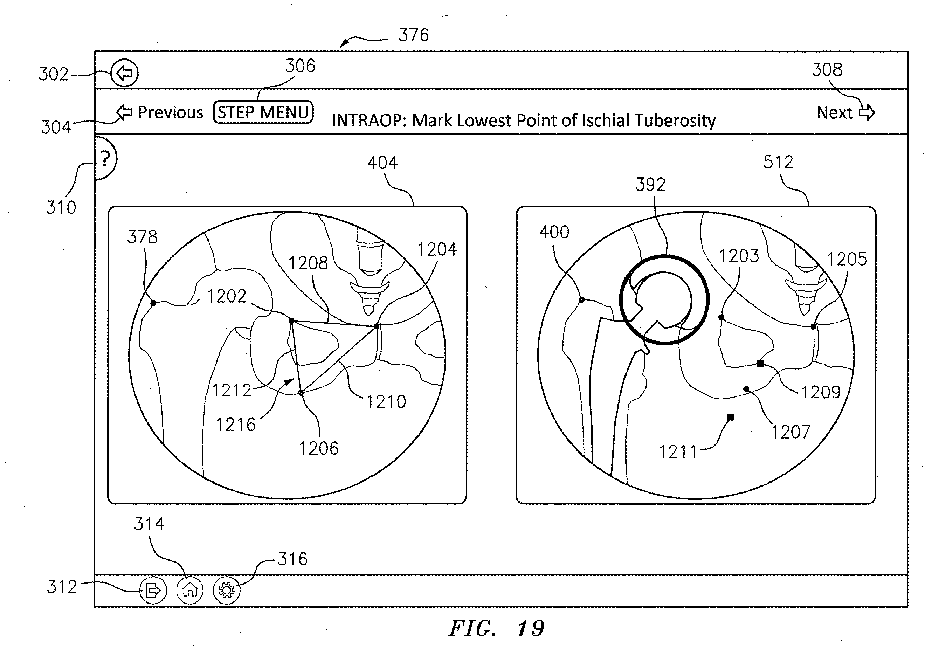

FIG. 19 is a schematic digital view screen 376 showing how Digital Annotation Module 3034, FIG. 1 may be used to optionally identify additional points on the pelvic anatomy for error analysis. Digital annotation point 1207 in intraop image 512 can be moved using digital handles 1209 and 1211 so that they mark the lowest point on the ischial tuberosity, similarly to point 1206 that has been positioned in preop image 404. Additional digital points 1202 and 1204 in preop image 504 have also been positioned in preop image 404 to draw the triangle created by digital lines 1208, 1210, and 1212. A similar triangle will be created in the intraop image 512 with the user completes the positioning of digital point 1207 by linking this position with additional digital annotations 1203 and 1205. In a later step, the triangles may be overlaid for visual comparison if desired, so that a system user can more easily identify any visual error.

In Step 4724, FIG. 11, Image Comparison Module 3030, FIG. 1 guides the positioning of a 'cutout' (exact copy) of the femur from the intraop image to create a digital overlay of the femur on the preop image. The system does this by connecting the preop image and the intraop 'cutout' of the femur based on the location of the greater trochanter annotations identified in Steps 4702 and 4710, FIG. 11. The system provides the user with the ability to rotate the femoral overlay image around the greater trochanter point. This enables the Module to record the change required to precisely align the preop and intraop femurs, which indicates the difference in femoral position, relative to the pelvic anatomy, caused by differences in femoral abduction.

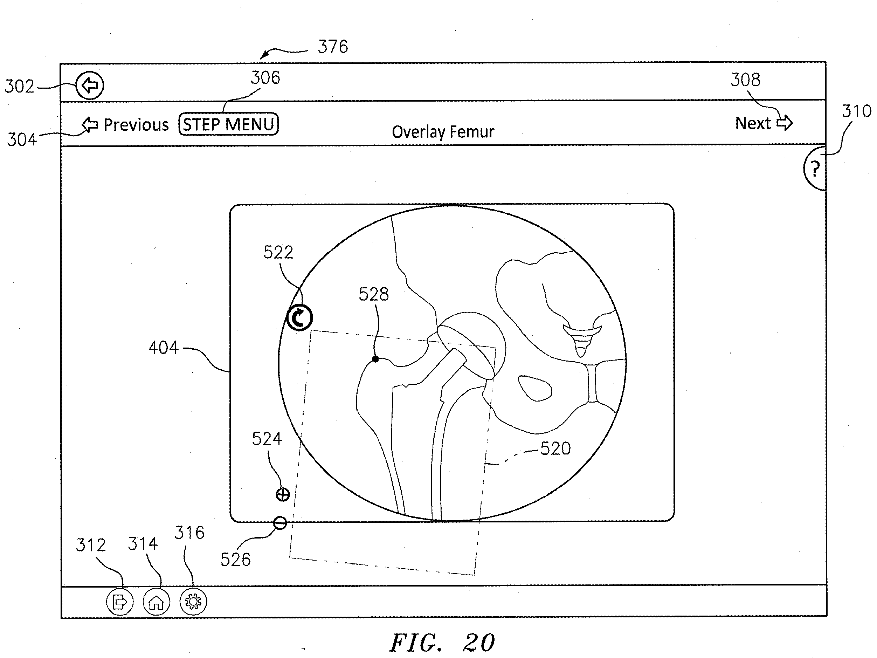

FIG. 20 is a schematic digital screen view 376 showing the placement of the intraop femoral 'cutout' 520 on top of preop image 404 by connecting the images at the greater trochanter annotation point 528, at which the preop and intraop greater trochanter points are collocated. Digital rotation handle 522 allows the user to manipulate the cutout, by rotating it around the immutable greater trochanter point 528 that connects the preop image 404 and femoral cutout 520. This enables the ability to align the femurs in the preop image and intraop cutout so that femoral anatomy is precisely aligned. Digital magnification handles 524 and 526 allow the user to increase or decrease the femoral cutout size, respectively, to account or correct for any differences in image scale.

Of note, Step 4724, FIG. 11 is not absolutely required to correct for femoral abduction, but is included in the preferred implementation according to the present invention because it facilitates the ability for a surgeon or other health care professional to visualize the correction required to account for femoral abduction. It is also a preferred implementation because it is extremely effective in picking up very subtle differences in femoral position due to differences in preop and intraop femoral abduction. Such subtle differences can be critical in precisely calculating leg length and offset in a later step, because a change in the results, due to femoral abduction, of even a few millimetres can adversely affect surgical outcomes and patient care. In alternative, but potentially less accurate implementations, this step will be entirely removed, and Image Comparison Module 3036, FIG. 1 will instead use the femoral axis tools that were positioned in Steps 4704 and 4714, FIG. 1 to calculate differences in femoral abduction. Because the images are now aligned by pelvic anatomy, any difference in angle between the positioned femoral axis tools in the preop and intraop images would indicate a difference due to femoral abduction.

In Step 4726, FIG. 11, Landmark Correction Module 3038, FIG. 1 calculates the difference in the femoral axis angle between the preop and intraop images using data gathered in the previous step. The terms "femoral angle" and "femoral axis angle" refer to the orientation of the longitudinal axis of the femur. If, for example, the preop and intraop femoral axis angle in relation to pelvic anatomy varies by 2 degrees, the difference calculated in this step will be 2 degrees. This step is necessary because any differences between the preop and intraop positioning of the femur, relative to the pelvis, make direct mathematical comparison of the greater trochanter position in space inaccurate. Therefore, this information will be used in Step 4732 to calculate an updated vector between the two femoral landmarks in the overlay that takes into account femoral abduction.

In Step 4728, FIG. 11, Landmark Correction Module 3038, FIG. 1 generates an additional "corrected" or "phantom" landmark point that accounts for any differences, when rendering the greater trochanter annotations in the preop and intraop images, in femoral position due to differences in femoral abduction between the images.

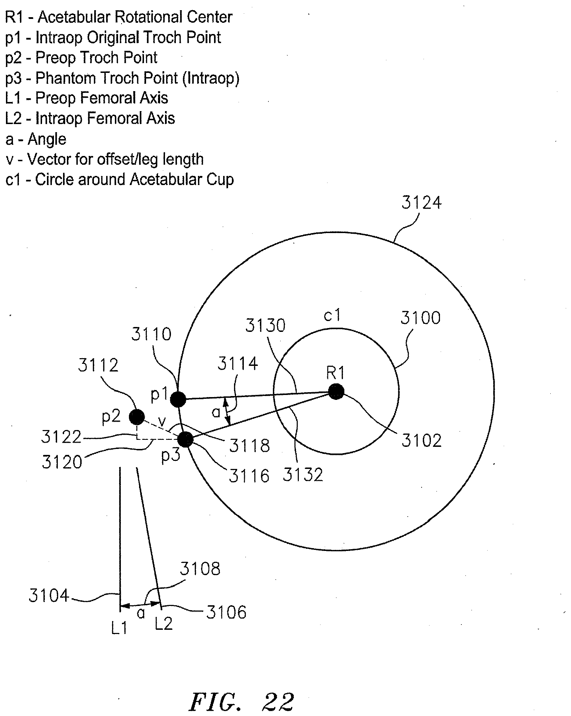

To generate the corrected landmark point in Step 4728, FIG. 11, the module first calculates $angle_{femur}$, which is the angular difference between the longitudinal axes of the femur in the preoperative and intraoperative images, respectively, also referred to as the preop and intraop femoral axis lines in the overlay. This technique is shown schematically in FIG. 22 for angle $\alpha$, arrow 3108, between longitudinal axis lines 3104 ("L1") and 3106 ("L2"). The system incorporates this with the femoral or acetabular center of rotation 3102 ("R1"), $(X_{origin}, Y_{origin})$ in the intraop image, previously identified in step 4712, FIG. 11, and the greater trochanter point 3110 ("p1"), $(X_{troch}, Y_{troch})$ in the intraop image. The system uses the following formulas to calculate the corrected landmark "phantom" point 3116 ("p3"), $(X_{phantom}, Y_{phantom})$ in Equations 1 and 2:

EQ. 1:

$$X_{phantom} = (X_{troch} - X_{origin}) * cosine(angle_{femur}) -$$
$$(Y_{troch} - Y_{origin}) * sine(angle_{femur}) + X_{origin}$$

EQ. 2:

$$Y_{phantom} = (X_{troch} - X_{origin}) * sine(angle_{femur}) +$$
$$(Y_{troch} - Y_{origin}) * cosine(angle_{femur}) + Y_{origin}$$

A vector "v", line 3118, is extended from the preoperative landmark point 3112 ("p2") to corrected landmark point 3116. Right triangle "legs" 3120 and 3122 are utilized to estimate offset and leg length, respectively. Leg 3122 is generally parallel to preoperative femoral axis 3104 in this construction. The Acetabular circle 3100 ("c1") assists in locating center of rotation 3102. Also shown in FIG. 21 are radius lines 3130 and 3132 which are also separated by angle $\alpha$, arrow 3114.

In Step 4730, FIG. 11, Image Comparison Module 3036, FIG. 18 superimposes the preop and intraop images by aligning pelvic anatomy, with the images displayed with some transparency so that both can be visualized in the overlay. In a preferred implementation the overlaid images will show the identified femoral landmarks placed on the greater trochanter so that location differences between the two points can be visualized, and will additionally show the corrected landmark point calculated in the previous Step 4728 that corrects for differences in femoral abduction between the two images. The system will maintain the location of the generated greater trochanter points and the femoral axis lines, relative to the preop and intraop images, as the images are manipulated to create the image overlay.

The Image Comparison Module 3036 can align the images according to pelvic anatomy in a variety of ways in this step. In preferred implementations of this system as described above, the system will have guided the user in identifying at least two consistent points on the pelvic anatomy in both images. The Image Comparison Module 3036 then superimposes the images so that the stationary base lines are positioned identically. The images are scaled, aligned and superimposed based on the pelvic reference lines drawn across consistent points on the pelvis in each image. The Image Comparison Module will move and scale all digital annotations in tandem with the underlying image so that they remain affixed to the underlying image. This includes positioning of the femoral and pelvic landmark annotations, the identified center of rotation of the femur, pelvic reference lines, the femoral axis lines, and any other annotations used in various constructions.

Alternative constructions obviate the need for the use of the pelvic reference lines. In one alternative construction, the system uses image recognition technique to autoidentify the pelvic anatomy and overlay the images based on the image recognition, then the user is presented with the option to manually manipulate the resulting overlay. In another alternative, the user will be guided to manually position the images so that the pelvic anatomy matches. The system in this method will provide the user with the ability to manipulate both the position of each of the images as well as adjust the magnification so that the pelvic anatomy can be superimposed on the overlay. Alternative systems will rely on hardware implementations and stationary cameras to obviate the need for a digital line, image recognition, or user manipulation whatsoever to create the overlay. In these instances, the external system may provide a known magnification ratio and the consistent patient positioning that would be required to create the image overlay without the use of pelvic reference lines or similar technique.

In Step 4732, FIG. 11, the Calculation Module 3040, FIG. 1, calculates the change in leg length and offset by analysing the vector between the greater trochanter point in the preop image and the calculated phantom point in the intraop image. To calculate leg length differential, the system calculates the distance between these two points along the longitudinal axis of the femur that was identified in the preop image. To calculate offset, the system calculates the distance between the two points along the axis that is perpendicular to the femoral axis from the preop image. All measurements are calculated in absolute measurement terms using the millimetre to pixel ratio calculated in a previous step.

Finally, in Step 4734, Output Module 3042, FIG. 1 generates an on-screen image of the results of this system. In a preferred implementation, the generated results will include an overlay of the preop and intraop image, along with the ability to visualize both of the digital annotation on the greater trochanters and additionally the generated phantom point that has been calculated in order to correct for differences in femoral abduction. Additionally, the system will show a calculated value for leg length, and possibly offset, which has taken into account the correction for femoral abduction.

FIG. 21 is a schematic digital screen 376 rendered by Output Module 3042, FIG. 1, showing one rendering of the preoperative and intraoperative images overlaid on one another, along with the leg length discrepancy calculated in Step 4732, FIG. 11, including the correction for differences in femoral abduction. Digital image frame 3070 contains an image of the intraop hip, with various digital annotations. The preop hip is contained within digital frame 3052. Preop and intraop images have been aligned according to pelvic reference line 3056, which corresponds to the pelvic reference line in both the preop and intraop images after the images have been aligned and scaled. Acetabular component AC is enclosed by circular annotation 3080 with center of rotation 3081. Point 3074 is the digital annotation point positioned on the intraop image, and is on line 3083, that provides a visualization of the circle on which the femoral point will move when the leg is abducted. Digital point 3082 is the phantom point which has been adjusted by the system based upon the original intraop greater trochanter point and the calculated difference in femoral rotation between the preop and intraop images. Digital point annotation 3054 identifies the greater trochanter in the preop image as previously positioned.

Toggle 806, FIG. 21, provides the user with the ability to turn the various circular and phantom landmarks on or off. Digital toggle icons 808 and 810 within collapsible control 812 allow the user to activate either the preop or intraop images, and rotate or move them in order to manually adjust the images if desired. When an image is activated, a rotation handle such as rotation handle 3099 will be generated and activate to modify the appropriate image frame. Digital buttons 800 provide the user with the ability to draw circular or linear annotation objects on top of the objects if desired. The calculated leg length discrepancy, identified as +3 mm intraoperative change within the collapsible frame identified by reference 3090, indicates a leg length that has gained 3 mm intraoperatively after correcting for any differences in femoral abduction between the preop and intraop images. Collapsible digital frame 3090 also shows information for a preoperative goal for leg length that may have been generated prior to the surgical procedure. In this particular screen depicted in FIG. 21, Preoperative Leg Length is listed as 'N/A', indicating that the surgeon did not set a preoperative leg length target prior to surgery. Legend 804 provides a legend that identifies the greater trochanter points on the preop and intraop images, as well as the point that has been adjusted to account for femoral abduction which is used for mathematical analysis of leg length. The Toggle icon 806 enables the user to show the femoral abduction correction point or otherwise turn it off so that the overlaid images are simply displayed. Digital frame 802, which can be opened by user touch, contains a transparency slider control that adjusts the relative transparency of preop image 3052 and intraop image 3070. The clickable command button labelled "<Previous", 304, allows the user to return to an earlier step, and the clickable "Next" button 308 completes the process and allows the user to complete and exit the system. Clickable "Step Menu" button 306 allows the user to return to any step in the system, so that data can be adjusted and the overlay recreated, if required.

Alternative constructions will change the order of various steps, including the generation of various digital-landmarks, while not materially altering the system and method. An additional alternative construction will correct for femoral abduction by generating a phantom point according to the greater trochanter annotation placement within the preop image rather than within the intraop image. To accomplish this would ideally rely upon identification of an estimated center of rotation in the preop image, rather than the intraop image center of rotation that is used in the preferred implementation described herein. Identifying the center of rotation in the preop image for such an alternative construction could be done by Digital Annotation Module 3034, FIG. 1, guiding placement of a digital circle placed around the femoral head, with a center at the estimated center of rotation.

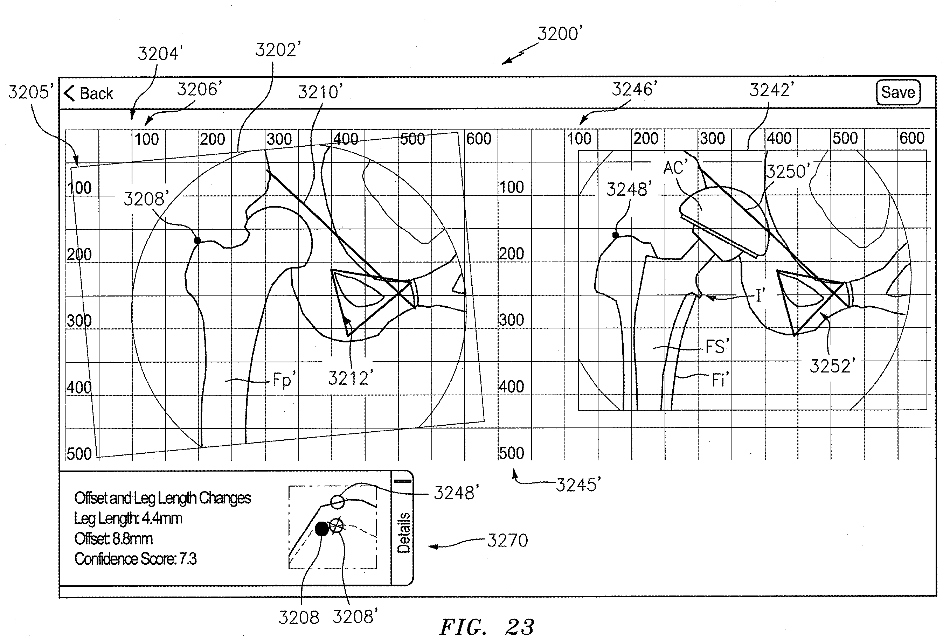

In yet another construction, reference (preop) and target (intraop) images are compared via a grid-type X-Y coordinate system without utilizing femoral angles, such as for preoperative images 3202' and intraoperative images 3242' in screen view 3200' illustrated in FIG. 23. The reference and intraoperative images are not actually digitally overlaid one on top of the other in this construction; instead, preop image 3202' is overlaid with, or otherwise associated with, a grid 3204' having a Y-axis 3205' and an X axis 3306' with units "100, 200, ...500" as shown, with the origin in the upper left-hand corner of grid 3204'. In a similar manner, intraop image 3242' is associated with a grid 3244' having a Y-axis 3245' and an X axis 3346'.

Preop image 3202', FIG. 23, includes femur Fp' with landmark point 3208' on the greater trochanter, and stationary base 3210' and error triangle 3212' on the pelvis. Intraop image 3242' includes femur Fi' with implant I' having femoral stem FS' and acetabular cup AC'. Intraoperative landmark point 3248' has been placed on the greater trochanter. Stationary base 3250' and error triangle 3253' have been placed on the pelvis.

After a user activates a "Proceed To Analysis" icon, the system aligns preop image 3202', FIG. 23, with intraop image 3242'. In this example, preop image 3202' has been "tilted" or rotated counter-clockwise relative to the initial position of preop image 3202 to represent alignment achieved using stationary base 3210' and 3250'. After both preop image and 3202' and 3242' have been aligned relative to each other, then a difference in position of one of the landmark points is determined, such as the shift of preop landmark point 3208 to the aligned position of preop landmark point 3208', FIG. 23. In this example, intraoperative landmark point 3248' is in the same grid location as intraoperative landmark point 3248. A vector can then be calculated from intraop landmark point 3248' to corrected point 3208' using calculations similar to that described above in relation to FIG. 22. In this construction, a "Details" window 3270 graphically shows the change in position of initial preop landmark point 3208 to corrected landmark point 3208'.

Other alternative constructions will change the order of various steps, including the generation of various digital landmarks. An additional alternative construction will identify an estimated center of rotation in the preop image instead of the intraop image, using a similar digital circle placed around the femoral head, or similar technique to annotate the estimate center of rotation.

Although specific features of the present invention are shown in some drawings and not in others, this is for convenience only, as each feature may be combined with any or all of the other features in accordance with the invention. While there have been shown, described, and pointed out fundamental novel features of the invention as applied to one or more preferred embodiments thereof, it will be understood that various omissions, substitutions, and changes in the form and details of the devices illustrated, and in their operation, may be made by those skilled in the art without departing from the scope of the invention, as defined by the claims. For example, it is expressly intended that all combinations of those elements and/or steps that perform substantially the same function, in substantially the same way, to achieve the same results be within the scope of the invention. Substitutions of elements from one described embodiment to another are also fully intended and contemplated.

It is also to be understood that the drawings are not necessarily drawn to scale, but that they are merely conceptual in nature.

**Claims**

1. A computer implemented method for accounting for femoral abduction, comprising:

   acquiring a preoperative image (404) of a surgical site, the surgical site including a skeletal bone and a femur, the femur capable of articulating with respect to the skeletal bone;
   acquiring an intraoperative image (512) of the surgical site after an implant has been affixed to the femur;
   generating a first landmark point (400; 528) on the femur in the intraoperative image;
   generating the first landmark point (378; 528) on the femur in the preoperative image;
   arranging a cutout (520) of the femur from the intraoperative image on the preoperative image, such that the first landmark points (528) are collocated;
   determining a difference angle ($\alpha$) about the collocated first landmark points between a femoral axis (L1; 3104) of the femur in the preoperative image relative to a femoral axis (L2; 3108) of the femur in the intraoperative cutout image;
   arranging the intraoperative image and the preoperative image, such that the skeletal bone in the intraoperative image and in the preoperative image are in overlying alignment with respect to each other;
   identifying a centre of rotation (3102) of the implant in at least one of the preoperative and intraoperative images;
   identifying a corrected landmark point (3116), wherein the corrected landmark point is equal to the first landmark point in the intraoperative image after it is rotated, about the centre of rotation of the implant, an amount equal to the difference angle; and
   calculating at least one of offset and length differential of at least the femur by analysing the vector (v) between the first landmark point in the preoperative image (3112) and the corrected landmark point (3116), thereby accounting for femoral abduction,
   wherein calculating the length differential of the femur comprises calculating the component of the vector (3122) along the femoral axis (3104) of the femur in the preoperative image, and
   wherein calculating the offset of the femur comprises calculating the component of the vector (3120) along a line perpendicular to the femoral axis (3104) of the femur in the preoperative image.

2. A system (141) including a memory, a user interface (3035) having a display (376) capable of providing at least visual guidance to a user of the system, and a processor, with the processor executing a program performing the steps of:

acquiring a preoperative image (404) of a surgical site, the surgical site including a skeletal bone and a femur, the femur capable of articulating with respect to the skeletal bone;

acquiring an intraoperative image (512) of the surgical site after an implant has been affixed to the femur;

generating a first landmark point (400; 528) on the femur in the intraoperative image;

generating the first landmark point (378; 528) on the femur in the preoperative image;

arranging a cutout (520) of the femur from the intraoperative image on the preoperative image, such that the first landmark points (528) are collocated;

determining a difference angle ($\alpha$) about the collocated first landmark points between a femoral axis of the femur in the preoperative image relative to a femoral axis of the femur in the intraoperative cutout image;

arranging the intraoperative image and the preoperative image, such that the skeletal bone in the intraoperative image and in the preoperative image are in overlying alignment with respect to each other;

identifying a centre of rotation (3102) of the implant in at least one of the preoperative and intraoperative images;

identifying a corrected landmark point (3116), wherein the corrected landmark point is equal to the first landmark point in the intraoperative image after it is rotated, about the centre of rotation of the implant, an amount equal to the difference angle; and

calculating at least one of offset and length differential of at least the femur by analysing the vector (v) between the first landmark point (3112) in the preoperative image and the corrected landmark point (3116), thereby accounting for femoral abduction,

wherein calculating the length differential of the femur comprises calculating the component of the vector (3122) along the femoral axis (3104) of the femur in the preoperative image, and

wherein calculating the offset of the femur comprises calculating the component of the vector (3120) along a line perpendicular to the femoral axis (3104) of the femur in the preoperative image.

3. The method of claim 1 or the system of claim 2, wherein the step of determining the difference angle comprises calculating the degree of rotation about the collocated first landmark points.

4. The method of claim 1 or the system of claim 2, further including the step of scaling the intraoperative image based on a known dimension of the implanted device.

5. The method of claim 1 or the system of claim 2, further including the step of scaling the preoperative image.

6. The method of claim 1 or the system of claim 2, further including the step of generating a reference line or a plurality of anatomical points on the first bone in the intraoperative and preoperative images to aid in overlaying or aligning the intraoperative and preoperative images.

7. The method of claim 1 or the system of claim 2, wherein the step of identifying the centre of rotation of the implant further includes generating a circle (3100) around a component of the implanted device, where that component operates as a ball in a ball and socket joint, and the center of the circle is identified as the centre of rotation of the implant.

8. The method of claim 1 or the system of claim 2, wherein the first landmark point is the greater trochanter.

9. The method of claim 1 or the system of claim 2, wherein the pelvis of the patient is selected as the skeletal bone, and the implant comprises an acetabular cup and a femoral stem having a shoulder and pivotally connectable to the acetabular cup.

**Patentansprüche**

1. Computerimplementiertes Verfahren zum Nachweisen einer femoralen Abduktion, umfassend:

Beziehen eines präoperativen Bildes (404) einer Operationsstelle, wobei die Operationsstelle einen Skelettknochen und einen Oberschenkelknochen (Femur) beinhaltet, wobei der Oberschenkelknochen in Bezug auf den Skelettknochen gelenkigfähig ist;

Erfassen eines intraoperativen Bildes (512) der Operationsstelle, nachdem ein Implantat an dem Oberschenkelknochen befestigt worden ist;

Generieren eines ersten Orientierungspunktes (400; 528) auf dem Oberschenkelknochen in dem intraoperativen Bild; Generieren des ersten Orientierungspunktes (378; 528) auf dem Oberschenkelknochen in dem präoperativen Bild;

Anordnen eines Ausschnitts (520) des Oberschenkelknochens aus dem intraoperativen Bild auf dem präoperativen Bild, sodass die ersten Orientierungspunkte (528) zusammenliegen;

Bestimmen eines Differenzwinkels ($\alpha$) um die zusammenliegenden ersten Orientierungspunkte zwischen einer Femurachse (L1; 3104) des Femurs in dem präoperativen Bild relativ zu einer Femurachse (L2; 3108) des Femurs in dem intraoperativen Ausschnittbild;

Anordnen des intraoperativen Bildes und des präoperativen Bildes, sodass sich der Skelettknochen in dem intraoperativen Bild und in dem präoperativen Bild in überlagernder Ausrichtung zueinander befinden;

Identifizieren eines Drehmittelpunkts (3102) des Implantats in wenigstens einem von dem präoperativen und dem intraoperativen Bild;

Identifizieren eines korrigierten Orientierungspunkts (3116), wobei der korrigierte Orientierungspunkt gleich dem ersten Orientierungspunkt in dem intraoperativen Bild ist, nachdem dieses um einen Betrag gleich dem Differenzwinkel um den Drehmittelpunkt des Implantats gedreht wurde; und

Berechnen wenigstens eines Versatzes und einer Längendifferenz von wenigstens dem Femur durch Analysieren des Vektors (v) zwischen dem ersten Orientierungspunkt (3112) in dem präoperativen Bild und dem korrigierten Orientierungspunkt (3116), wodurch eine femorale Abduktion nachgewiesen wird,

wobei das Berechnen der Längendifferenz des Femurs das Berechnen der Komponente des Vektors (3122) entlang der Femurachse (3104) des Femurs in dem präoperativen Bild umfasst und

wobei das Berechnen des Versatzes des Femurs das Berechnen der Komponente des Vektors (3120) entlang eine Linie umfasst, die senkrecht zur Femurachse (3104) des Femurs in dem präoperativen Bild verläuft.

2. System (141), aufweisend einen Speicher, eine Benutzerschnittstelle (3035) mit einem Display (376), die in der Lage ist, wenigstens eine visuelle Anleitung für einen Benutzer des Systems bereitzustellen, und einen Prozessor, wobei der Prozessor ein Programm ausführt, dass diese Schritte durchführt:

Beziehen eines präoperativen Bildes (404) einer Operationsstelle, wobei die Operationsstelle einen Skelettknochen und einen Oberschenkelknochen (Femur) beinhaltet, wobei der Oberschenkelknochen in Bedzug auf den Skelettknochen gelenkigfähig ist;

Erfassen eines intraoperativen Bildes (512) der Operationsstelle, nachdem ein Implantat an dem Oberschenkelknochen befestigt worden ist;

Generieren eines ersten Orientierungspunktes (400; 528) auf dem Oberschenkelknochen in dem intraoperativen Bild; Generieren des ersten Orientierungspunktes (378; 528) auf dem Oberschenkelknochen in dem präoperativen Bild;

Anordnen eines Ausschnitts (520) des Oberschenkelknochens aus dem intraoperativen Bild auf dem präoperativen Bild, sodass die ersten Orientierungspunkte (528) zusammenliegen;

Bestimmen eines Differenzwinkels ($\alpha$) um die zusammenliegenden ersten Orientierungspunkte zwischen einer Femurachse des Femurs in dem präoperativen Bild relativ zu einer Femurachse des Femurs in dem intraoperativen Ausschnittbild;

Anordnen des intraoperativen Bildes und des präoperativen Bildes, sodass sich der Skelettknochen in dem intraoperativen Bild und in dem präoperativen Bild in überlagernder Ausrichtung zueinander befinden;

Identifizieren eines Drehmittelpunkts (3102) des Implantats in wenigstens einem von dem präoperativen und dem intraoperativen Bild;

Identifizieren eines korrigierten Orientierungspunkts (3116), wobei der korrigierte Orientierungspunkt gleich dem ersten Orientierungspunkt in dem intraoperativen Bild ist, nachdem dieses um einen Betrag gleich dem Differenzwinkel um den Drehmittelpunkt des Implantats gedreht wurde; und

Berechnen wenigstens eines Versatzes und einer Längendifferenz von wenigstens dem Femur durch Analysieren des Vektors (v) zwischen dem ersten Orientierungspunkt (3112) in dem präoperativen Bild und dem korrigierten Orientierungspunkt (3116), wodurch eine femorale Abduktion nachgewiesen wird,

wobei das Berechnen der Längendifferenz des Femurs das Berechnen der Komponente des Vektors (3122) entlang der Femurachse (3104) des Femurs in dem präoperativen Bild umfasst und

wobei das Berechnen des Versatzes des Femurs das Berechnen der Komponente des Vektors (3120) entlang eine Linie umfasst, die senkrecht zur Femurachse (3104) des Femurs in dem präoperativen Bild verläuft.

3. Verfahren nach Anspruch 1 oder System nach Anspruch 2, wobei der Schritt des Bestimmens des Differenzwinkels das Berechnen des Drehungsgrades um die zusammenliegenden ersten Orientierungspunkte umfasst.

**4.** Verfahren nach Anspruch 1 oder System nach Anspruch 2, ferner aufweisend den Schritt des Skalierens des intraoperativen Bildes basierend auf einer bekannten Abmessung der implantierten Vorrichtung.

**5.** Verfahren nach Anspruch 1 oder System nach Anspruch 2, ferner aufweisend den Schritt des Skalierens des präoperativen Bildes.

**6.** Verfahren nach Anspruch 1 oder System nach Anspruch 2, ferner aufweisend den Schritt des Generierens einer Referenzlinie oder mehrerer anatomischer Punkte auf dem ersten Knochen in dem intraoperativen Bild und dem präoperativen Bild, um das Überlagern oder Ausrichten des intraoperativen und des präoperativen Bildes zu unterstützen.

**7.** Verfahren nach Anspruch 1 oder System nach Anspruch 2, wobei der Schritt des Identifizierens des Drehmittelpunktes des Implantats ferner das Generieren eines Kreises (3100) um eine Komponente des implantierten Vorrichtung beinhaltet, wobei diese Komponente als Kugel in einem Kugelgelenk fungiert, und die Mitte des Kreises als der Drehmittelpunkt des Implantats identifiziert ist.

**8.** Verfahren nach Anspruch 1 oder System nach Anspruch 2, wobei der erste Orientierungspunkt der große Rollhügel (Trochanter) ist.

**9.** Verfahren nach Anspruch 1 oder System nach Anspruch 2, wobei das Becken des Patienten als Skelettknochen ausgewählt ist und das Implantat eine Hüftpfanne und einen Oberschenkelschaft mit einer Schulter aufweist und drehbar mit der Hüftpfanne verbunden werden kann.

**Revendications**

**1.** Procédé mis en œuvre par ordinateur pour la prise en compte de l'abduction fémorale, comprenant :

l'acquisition d'une image préopératoire (404) d'un site chirurgical, le site chirurgical comportant un os du squelette et un fémur, le fémur pouvant s'articuler par rapport à l'os du squelette ;
l'acquisition d'une image intra-opératoire (512) du site chirurgical après qu'un implant a été fixé au fémur ;
la génération d'un premier point de repère (400 ; 528) sur le fémur dans l'image intra-opératoire ;
la génération du premier point de repère (378 ; 528) sur le fémur dans l'image préopératoire ;
l'agencement d'un découpage (520) du fémur issu de l'image intra-opératoire sur l'image préopératoire, de telle sorte que les premiers points de repère (528) soient colocalisés ;
la détermination d'un angle différentiel ($\alpha$) autour des premiers points de repère colocalisés entre un axe fémoral (L1 ; 3104) du fémur dans l'image préopératoire et un axe fémoral (L2 ; 3108) du fémur dans l'image intra-opératoire découpée ;
l'agencement de l'image intra-opératoire et de l'image préopératoire de telle sorte que l'os du squelette dans l'image intra-opératoire et l'os du squelette dans l'image préopératoire soient en alignement superposé l'un par rapport à l'autre ;
l'identification d'un centre de rotation (3102) de l'implant dans au moins une des images préopératoire et intra-opératoire ;
l'identification d'un point de repère corrigé (3116), le point de repère corrigé étant égal au premier point de repère dans l'image intra-opératoire après qu'elle a été tournée autour du centre de rotation de l'implant d'une quantité égale à l'angle différentiel ; et
le calcul d'au moins un paramètre parmi un décalage et un différentiel de longueur au moins du fémur par analyse du vecteur (v) entre le premier point de repère dans l'image préopératoire (3112) et le point de repère corrigé (3116), pour prendre ainsi en compte l'abduction fémorale ;
dans lequel le calcul du différentiel de longueur du fémur comprend le calcul de la composante du vecteur (3122) le long de l'axe fémoral (3104) du fémur dans l'image préopératoire, et
dans lequel le calcul du décalage du fémur comprend le calcul de la composante du vecteur (3120) le long d'une ligne perpendiculaire à l'axe fémoral (3104) du fémur dans l'image préopératoire.

**2.** Système (141) comportant une mémoire, une interface utilisateur (3035) ayant un écran (376) pouvant fournir au moins des indications visuelles à un utilisateur du système, et un processeur, le processeur exécutant un programme effectuant les étapes suivantes :

acquisition d'une image préopératoire (404) d'un site chirurgical, le site chirurgical comportant un os du squelette et un fémur, le fémur pouvant s'articuler par rapport à l'os du squelette ;

acquisition d'une image intra-opératoire (512) du site chirurgical après qu'un implant a été fixé au fémur ;

génération d'un premier point de repère (400 ; 528) sur le fémur dans l'image intra-opératoire ;

génération du premier point de repère (378 ; 528) sur le fémur dans l'image préopératoire ;

l'agencement d'un découpage (520) du fémur issu de l'image intra-opératoire sur l'image préopératoire, de telle sorte que les premiers points de repère (528) soient colocalisés ;

détermination d'un angle différentiel ($\alpha$) autour des premiers points de repère colocalisés entre un axe fémoral du fémur dans l'image préopératoire et un axe fémoral du fémur dans l'image intra-opératoire découpée ;

l'agencement de l'image intra-opératoire et de l'image préopératoire de telle sorte que l'os du squelette dans l'image intra-opératoire et l'os du squelette dans l'image préopératoire soient en alignement superposé l'un par rapport à l'autre ;

l'identification d'un centre de rotation (3102) de l'implant dans au moins une des images préopératoire et intra-opératoire ;

identification d'un point de repère corrigé (3116), le point de repère corrigé étant égal au premier point de repère dans l'image intra-opératoire après qu'elle a été tournée autour du centre de rotation de l'implant d'une quantité égale à l'angle différentiel ; et

calcul d'au moins un paramètre parmi un décalage et un différentiel de longueur au moins du fémur par analyse du vecteur (v) entre le premier point de repère (3112) dans l'image préopératoire et le point de repère corrigé (3116), pour prendre ainsi en compte l'abduction fémorale ;

dans lequel le calcul du différentiel de longueur du fémur comprend le calcul de la composante du vecteur (3122) le long de l'axe fémoral (3104) du fémur dans l'image préopératoire, et

dans lequel le calcul du décalage du fémur comprend le calcul de la composante du vecteur (3120) le long d'une ligne perpendiculaire à l'axe fémoral (3104) du fémur dans l'image préopératoire.

3. Procédé selon la revendication 1 ou système selon la revendication 2, dans lesquels l'étape de détermination de l'angle différentiel comprend le calcul du degré de rotation autour des premiers points de repère colocalisés.

4. Procédé selon la revendication 1 ou système selon la revendication 2, comportant en outre l'étape de mise à l'échelle de l'image intra-opératoire sur la base d'une dimension connue du dispositif implanté.

5. Procédé selon la revendication 1 ou système selon la revendication 2, comportant en outre l'étape de mise à l'échelle de l'image préopératoire.

6. Procédé selon la revendication 1 ou système selon la revendication 2, comportant en outre l'étape de génération d'une ligne de référence ou d'une pluralité de points anatomiques sur le premier os dans les images intra-opératoire et préopératoire pour faciliter la superposition ou l'alignement des images intra-opératoire et préopératoire.

7. Procédé selon la revendication 1 ou système selon la revendication 2, dans lesquels l'étape d'identification du centre de rotation de l'implant comporte en outre la génération d'un cercle (3100) autour d'un composant du dispositif implanté, où ce composant fonctionne comme une rotule dans une articulation à rotule, et le centre du cercle est identifié comme le centre de rotation de l'implant.

8. Procédé selon la revendication 1 ou système selon la revendication 2, dans lesquels le premier point de repère est le grand trochanter.

9. Procédé selon la revendication 1 ou système selon la revendication 2, dans lesquels le bassin du patient est sélectionné comme l'os du squelette, et l'implant comprend une coupe acétabulaire et une tige fémorale ayant un épaulement et raccordable avec faculté de pivotement à la coupe acétabulaire.

<u>3028</u>

3032

3030 ── Image Capture Module

Image Scaling and Alignment Module

3034 ── Digital Annotation Module

UI ── 3035

3042

Output Module

3036 ── Image Comparison Module

3038 ── Landmark Correction Module

3040 ── Calculation Module

── 3037

*FIG. 1*

FIG. 2

FIG. 3

*FIG. 4*

FIG. 5

EP 3 402 409 B1

**FIG. 6**

FIG. 7

FIG. 8

EP 3 402 409 B1

FIG. 9

EP 3 402 409 B1

FIG. 10

Start ) Flowchart OA

Scale and Align Images —— 4718

**Preop Hip**

Capture Preop Image —— 4700

Identify Greater Trochanter —— 4702

Identify Longitudinal Axis of Femur —— 4704

Draw Pelvic Reference Line —— 4706

**Intraop Hip**

Capture Intraop Image —— 4708

Identify Greater Trochanter —— 4710

Draw Circle Around Acetabular Component and Enter Known Size of Component —— 4712

Identify Longitudinal Axis of Femur —— 4714

Draw Pelvic Reference Line —— 4716

Include Error Analysis?

YES

4722

Generate Error Analysis Point(s) on Pelvic Anatomy

No    4720

Overlay Femurs —— 4724

**Femoral Abduction Correction**

4726 — Calculate Difference in Femoral Axis Angle Between Preop and Intraop Image

4728 — Generate Corrected Landmark Point in Intraop Image using Landmark Point and Difference in Femoral Axes

4730 — Create Image Overlay of Preop and Intraop Images

4732 — Calculate Change in Leg Length and Offset

Display Visual Output of Image Overlay with Leg Length Differential

End    4734

*FIG. 11*

31

*FIG. 12*

*FIG. 13*

**FIG. 14**

EP 3 402 409 B1

*FIG. 15*

EP 3 402 409 B1

FIG. 16

FIG. 17

EP 3 402 409 B1

FIG. 18

FIG. 19

EP 3 402 409 B1

302

306

308

310

304 ← Previous STEP MENU | Overlay Femur | Next ⇨

?

522

528

404

520

524

⊕

⊖

526

312 314 316

EP 3 402 409 B1

*FIG. 20*

*FIG. 21*

R1 - Acetabular Rotational Center
p1 - Intraop Original Troch Point
p2 - Preop Troch Point
p3 - Phantom Troch Point (Intraop)
L1 - Preop Femoral Axis
L2 - Intraop Femoral Axis
a - Angle
v - Vector for offset/leg length
c1 - Circle around Acetabular Cup

*FIG. 22*

FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014127354 A1
- US 63030015

- US 20150238271 A